(19) Europäisches Patentamt / European Patent Office / Office européen des brevets

(11) EP 3 890 703 B1

(12) **EUROPEAN PATENT SPECIFICATION**

(45) Date of publication and mention of the grant of the patent:
**06.09.2023 Bulletin 2023/36**

(21) Application number: **19821281.3**

(22) Date of filing: **04.12.2019**

(51) International Patent Classification (IPC):
***A61K 9/00*** *(2006.01)* ***A61K 9/08*** *(2006.01)*
***A61K 9/19*** *(2006.01)* ***A61K 47/10*** *(2017.01)*
***A61K 47/12*** *(2006.01)* ***A61K 47/18*** *(2017.01)*
***A61K 47/26*** *(2006.01)* ***A61K 31/5377*** *(2006.01)*
***A61K 47/32*** *(2006.01)* ***A61K 47/38*** *(2006.01)*

(52) Cooperative Patent Classification (CPC):
**A61K 9/0019; A61K 9/08; A61K 9/19; A61K 31/5377; A61K 47/10; A61K 47/12; A61K 47/183; A61K 47/26; A61K 47/32; A61K 47/38**

(86) International application number:
**PCT/IB2019/060437**

(87) International publication number:
**WO 2020/115676 (11.06.2020 Gazette 2020/24)**

(54) **NOVEL PHARMACEUTICAL FORMULATION COMPRISING A STING MODULATOR**

NEUARTIGE PHARMAZEUTISCHE FORMULIERUNG BEINHALTEND EINEN STING MODULATOR

NOUVELLE FORMULATION PHARMACEUTIQUE COMPRENANT UN MODULATEUR DU STING

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.12.2018 US 201862776102 P**

(43) Date of publication of application:
**13.10.2021 Bulletin 2021/41**

(73) Proprietor: **GlaxoSmithKline Intellectual Property Development Ltd**
**Brentford Middlesex TW8 9GS (GB)**

(72) Inventors:
- **DHANIKULA, Anand Babu**
  **Collegeville, Pennsylvania 19426 (US)**
- **MCQUEEN, Lisa**
  **Collegeville, Pennsylvania 19426 (US)**

(74) Representative: **Knight, Lucie Viktoria et al**
**GlaxoSmithKline**
**Global Patents (CN925.1)**
**980 Great West Road**
**Brentford, Middlesex TW8 9GS (GB)**

(56) References cited:
**WO-A1-2017/175147**

- **SERAJUDDIN ET AL: "Salt formation to improve drug solubility", ADVANCED DRUG DELIVERY REVIEWS, ELSEVIER, AMSTERDAM, NL, vol. 59, no. 7, 24 August 2007 (2007-08-24), pages 603-616, XP022211982, ISSN: 0169-409X, DOI: 10.1016/J.ADDR.2007.05.010**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 890 703 B1

## Description

### FIELD OF THE INVENTION

The present invention relates to a formulation and process of producing an injectable dosage form comprising (*E*)-1-(4-(5-Carbamoyl-2-(1-ethyl-3-methyl-1H-pyrazole-5-carboxamido)-7-(3-morpholinopropoxy)-1H-benzo[d]imidazol-1-yl)but-2-en-1-yl)-2-(1-ethyl-3-methyl-1H-pyrazole-5-carboxamido)-7-methoxy-1H-benzo[d]imidazole-5-carboxamide, represented by the following structure and hereinafter referred to as Compound A:

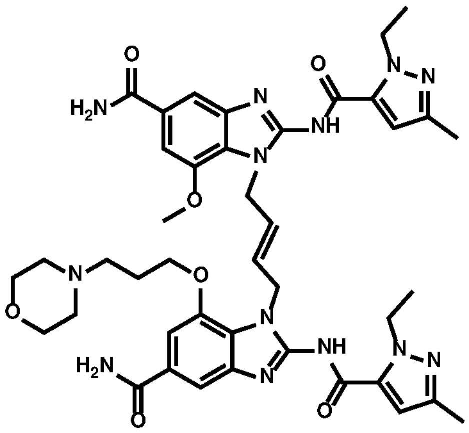
(Compound A),

or a tautomer thereof,
or a pharmaceutically acceptable salt thereof.

The invention particularly relates to a method of producing an aqueous, powder or lyophile pharmaceutical formulation. The aqueous formulation is ready to inject while the powder and lyophile formulations require reconstitution in a suitable media prior to administration.

### BACKGROUND OF THE INVENTION

(*E*)-1-(4-(5-Carbamoyl-2-(1-ethyl-3-methyl-1H-pyrazole-5-carboxamido)-7-(3-morpholinopropoxy)-1H-benzo[d]imidazol-1-yl)but-2-en-1-yl)-2-(1-ethyl-3-methyl-1H-pyrazole-5-carboxamido)-7-methoxy-1H-benzo[d]imidazole-5-carboxamide is a compound which is disclosed and claimed, along with tautomers, pharmaceutically acceptable salts, and solvates, including hydrates, thereof, as being useful as a modulator of transmembrane protein 173 (TMEM173), which is also known as STING (Stimulator of Interferon Genes), and particularly in the treatment of inflammation, allergic and autoimmune diseases, infectious diseases, a variety of cancers, pre-cancerous syndromes and as a vaccine adjuvant, in International Patent Application No. PCT/IB2017/051945, having an International filing date of April 5, 2017, an International Patent Application Publication Number WO 2017/175147 and an International Publication date of October 12, 2017.

The systemic administration of Compound A for the treatment of diseases in which modulation of STING (Stimulator of Interferon Genes) is beneficial, for example inflammation, allergic and autoimmune diseases, infectious diseases, hepatitis C virus (HCV), hepatitis B virus (HBV), influenza, skin warts, multiple sclerosis, human immunodeficiency virus (HIV) infection, AIDS, cancer, pre-cancerous syndromes and as immunogenic composition or vaccine adjuvant is disclosed in International Patent Application No. PCT/IB2018/057738, having an International filing date of October 4, 2018, an International Patent Application Publication Number WO 2019/069275 and International Publication date of April 11, 2019.

Pharmaceutical dosage forms are popular and useful forms of medications for dispensing pharmaceutically active compounds. A variety of such forms are known, including tablets, capsules, pellets, lozenges, and powders.

However, the formulation of an acceptable pharmaceutical dosage form on a commercial scale is not always straightforward. The formula and process of manufacture must be such as to provide an integral dosage form that maintains its integrity until used. Pharmaceutically active compounds with high molecular weight, low solubility and poor oral bioavailability can present particular challenges in preparing high quality dosage forms, since the physical properties of the drug influence the properties of the dosage form. The formulator must balance the drug's unique properties with the properties of each excipient in order to prepare a safe, efficacious and easy to use pharmaceutical dosage form.

Compound A is a high molecular weight compound (MW 849.937) that presents the formulator with unique concerns when attempting to formulate this compound into a suitable dosage form, suitably for systemic administration, with a desirable pharmacokinetic profile. Such concerns include but are not limited to, the tendency of the salts and solvates of Compound A to be highly hygroscopic, Compound A has poor bioavailability, and is minimally soluble in water (solubility is less than 0.01mg/mL). Significant realization of these concerns will result in difficulties in formulating Compound A.

It would be desirable to provide Compound A in a pharmaceutical dosage form on a commercial scale with a desirable pharmacokinetic profile.

## SUMMARY OF THE INVENTION

The invention relates to a pharmaceutical formulation comprising:

a) a pharmaceutically effective amount of Compound A having the structure

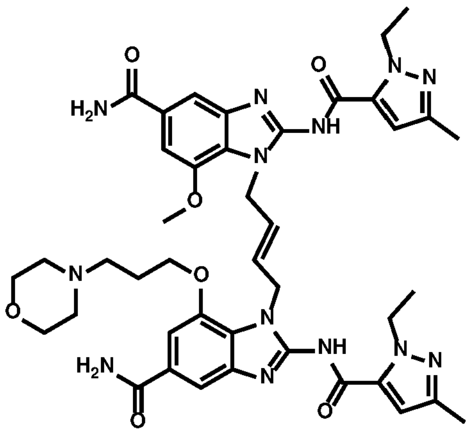

,

or a tautomer thereof,
or a pharmaceutically acceptable salt thereof;

b) a bulking agent or combination of bulking agents; and
c) a solubilizing agent selected from: aspartic acid, acetic acid, glutamic acid, methane sulfonic acid, lactic acid, and glucuronic acid.

In a specific embodiment of the invention, the formulation is prepared as a solution in water that comprises 2 mg of compound A, 96 mg mannitol, and 5.32 mg of L-aspartic acid. The solution is subsequently filled in a vial and lyophilized to a powder or cake. The lyophilized powder or cake is then reconstituted for use by dilution with a diluent, suitably water.

In a specific embodiment of the invention, the formulation is prepared as a solution in water that comprises 1 mg of compound A, 48 mg mannitol, and 2.66 mg of L-aspartic acid. The solution is subsequently filled in a vial and lyophilized to a powder or cake. The lyophilized powder or cake is then reconstituted for use by dilution with a diluent, suitably water.

In a specific embodiment of the invention, the formulation is prepared as a solution in water that comprises about 0.5 mg of compound A, about 48 mg mannitol, and 1.3 mg of L-aspartic acid. The solution is subsequently filled in a vial and lyophilized to a powder or cake. The lyophilized powder or cake is then reconstituted for use by dilution with a diluent, suitably water.

Another aspect of the invention is related to altering the drug concentration from 0.1 mg/mL to 4 mg/mL in water by adjusting the pH with suitable concentration of aspartic acid and mannitol for isotonicity. The solution can be subsequently filled into a vial and lyophilized into a powder or cake for reconstitution. Further, the concentration of drug in reconstitution medium can be altered by the volume of reconstitution medium added to the lyophilized powder or cake.

Another aspect of this invention relates to a composition for use in a method of treating a disease state selected from: inflammation, allergic and autoimmune diseases, infectious diseases, hepatitis C virus (HCV), hepatitis B virus (HBV), influenza, skin warts, multiple sclerosis, human immunodeficiency virus (HIV) infection, AIDS, cancer, and pre-cancerous syndromes, which method comprises administering to a patient in need thereof a therapeutically effective

amount of a pharmaceutical dosage form of the present invention.

Another aspect of this invention relates to a composition for use in a method of treating cancer which method comprises administering to a patient in need thereof a therapeutically effective amount of a pharmaceutical dosage form of the present invention.

## DETAILED DESCRIPTION OF THE INVENTION

The present invention is directed to injectable, suitably lyophilized, pharmaceutical compositions that are safe and stable, which are particularly useful for delivering Compound A to patients in need thereof.

In one embodiment, the invention relates to a pharmaceutical formulation comprising:

a) a pharmaceutically effective amount of Compound A having the structure

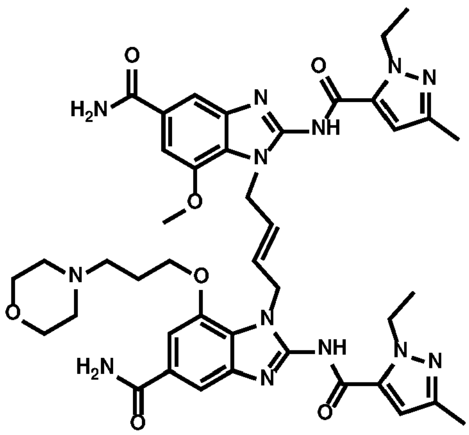
.

or a tautomer thereof,
or a pharmaceutically acceptable salt thereof;

b) a bulking agent or combination of bulking agents;
c) a solubilizing agent selected from: aspartic acid, acetic acid, glutamic acid, methane sulfonic acid, lactic acid, and glucuronic acid, in an amount effective to provide a pH between about 2 and 4; and optionally
d) water.

In one embodiment, the invention relates to a pharmaceutical formulation comprising:

a) a pharmaceutically effective amount of Compound A having the structure

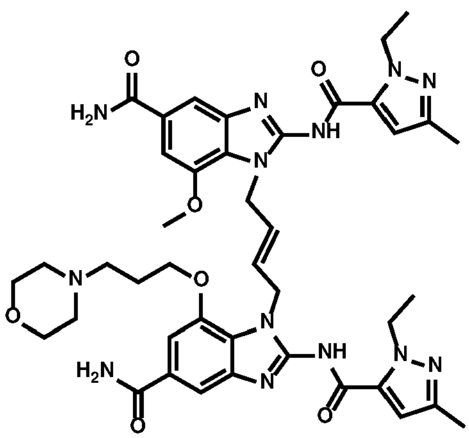
,

or a tautomer thereof,
or a pharmaceutically acceptable salt thereof;

b) a bulking agent or combination of bulking agents;
c) a solubilizing agent selected from: aspartic acid, acetic acid, glutamic acid, methane sulfonic acid, lactic acid, and glucuronic acid, in an amount effective to provide a pH between about 2.7 and 3.6; and optionally
d) water.

In one embodiment, the invention relates to a pharmaceutical formulation comprising:

a) a pharmaceutically effective amount of Compound A having the structure

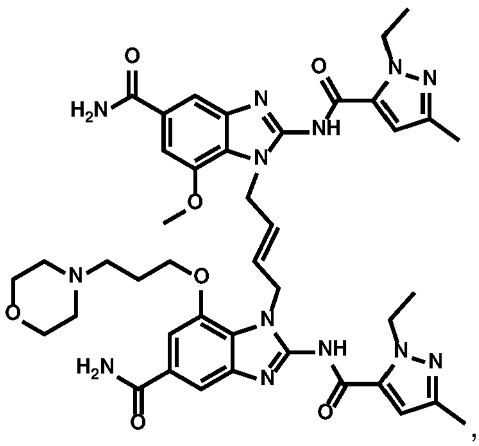

or a tautomer thereof,
or a pharmaceutically acceptable salt thereof;

b) a bulking agent or combination of bulking agents;
c) a solubilizing agent selected from: aspartic acid, acetic acid, glutamic acid, methane sulfonic acid, lactic acid, and glucuronic acid, in an amount effective to provide a pH between about 2.9 and 3.5; and optionally
d) water.

In one embodiment, the invention relates to a pharmaceutical formulation comprising:

a) a pharmaceutically effective amount of Compound A having the structure

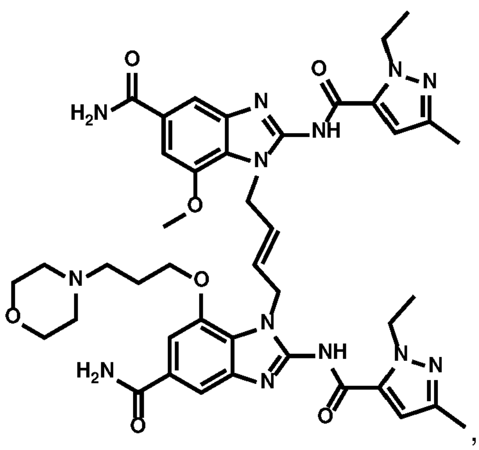

or a tautomer thereof,
or a pharmaceutically acceptable salt thereof;

b) a bulking agent or combination of bulking agents;
c) a solubilizing agent selected from: aspartic acid, acetic acid, glutamic acid, methane sulfonic acid, lactic acid,

and glucuronic acid, in an amount effective to provide a pH between about 3.0 and 3.5; and optionally
d) water.

In one embodiment, the invention relates to a pharmaceutical formulation comprising:

a) a pharmaceutically effective amount of Compound A having the structure

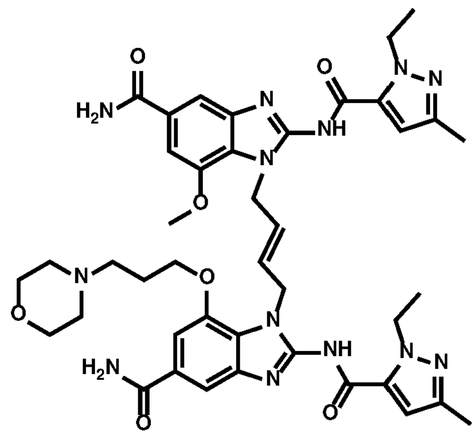

b) a pharmaceutically acceptable amount of a solubilizing agent effective to provide a pH between about 2 and 4; and
c) a pharmaceutically acceptable amount of a bulking agent, such as mannitol, and optionally forming a lyophilized cake.

In one embodiment, the invention relates to a pharmaceutical formulation comprising:

a) a pharmaceutically effective amount of Compound A having the structure

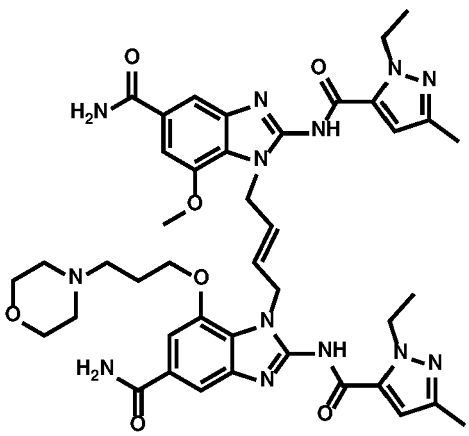,

or a tautomer thereof,
or a pharmaceutically acceptable salt thereof;

b) a bulking agent or combination of bulking agents; and
c) a solubilizing agent selected from: aspartic acid, acetic acid, glutamic acid, methane sulfonic acid, lactic acid, and glucuronic acid, in a molar ratio of Compound A:solubilizing agent from about 1:1 to 1:125; and optionally
d) water.

In one embodiment, the invention relates to a pharmaceutical formulation comprising:

a) a pharmaceutically effective amount of Compound A having the structure

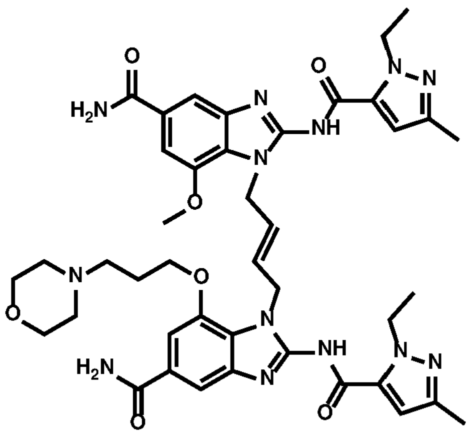

,

or a tautomer thereof,
or a pharmaceutically acceptable salt thereof;

b) a bulking agent or combination of bulking agents; and
c) a solubilizing agent selected from: aspartic acid, acetic acid, glutamic acid, methane sulfonic acid, lactic acid, and glucuronic acid, in a molar ratio of Compound A:solubilizing agent from about 1:1 to 1:30; and optionally
d) water.

In one embodiment, the invention relates to a pharmaceutical formulation comprising:

a) a pharmaceutically effective amount of Compound A having the structure

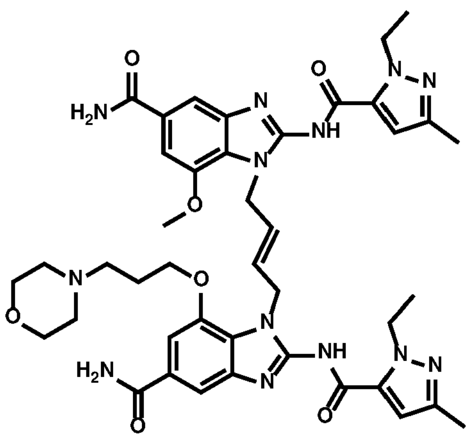

,

or a tautomer thereof,
or a pharmaceutically acceptable salt thereof;

b) a bulking agent or combination of bulking agents; and
c) a solubilizing agent selected from: aspartic acid, acetic acid, glutamic acid, methane sulfonic acid, lactic acid, and glucuronic acid, in a molar ratio of Compound A:solubilizing agent from about 1:1 to 1:100; and optionally
d) water.

In one embodiment, the invention relates to a pharmaceutical formulation comprising:

a) a pharmaceutically effective amount of Compound A having the structure

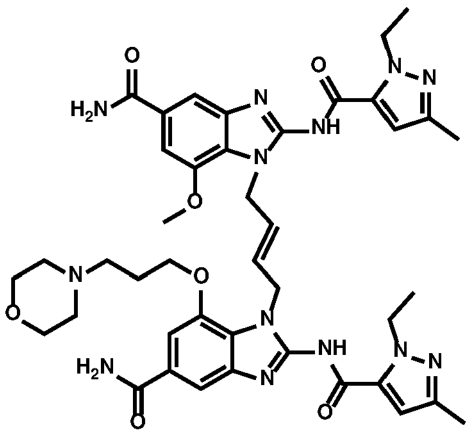,

or a tautomer thereof,
or a pharmaceutically acceptable salt thereof;

b) a bulking agent or combination of bulking agents; and
c) a solubilizing agent selected from: aspartic acid, acetic acid, glutamic acid, methane sulfonic acid, lactic acid, and glucuronic acid, in a molar ratio of Compound A:solubilizing agent from about 1:10 to 1:30; and optionally
d) water.

In one embodiment, the invention relates to a pharmaceutical formulation comprising:

a) a pharmaceutically effective amount of Compound A having the structure

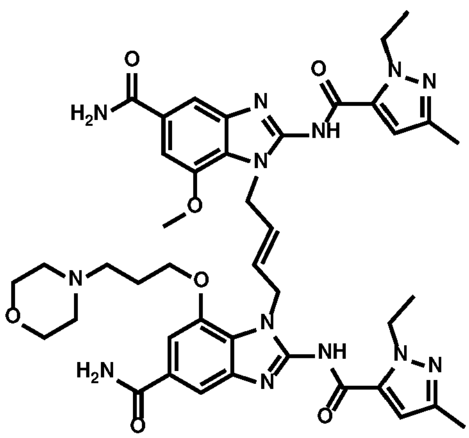,

or a tautomer thereof,
or a pharmaceutically acceptable salt thereof;

b) a bulking agent or combination of bulking agents; and
c) a solubilizing agent selected from: aspartic acid, acetic acid, glutamic acid, methane sulfonic acid, lactic acid, and glucuronic acid, in an amount from about 0.3 mg to about 4 mg per 1.0 mg of Compound A.

In one embodiment, the invention relates to a pharmaceutical formulation comprising:

a) a pharmaceutically effective amount of Compound A having the structure

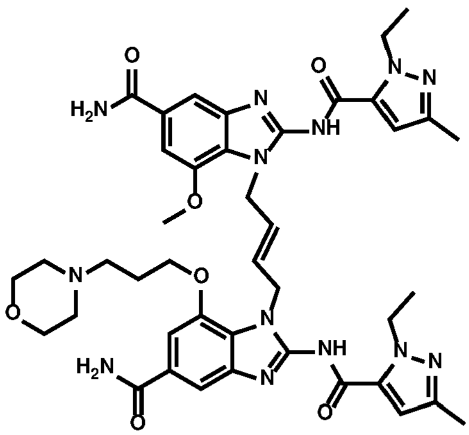

,

or a tautomer thereof,
or a pharmaceutically acceptable salt thereof;

b) a bulking agent or combination of bulking agents; and
c) aspartic acid in an amount from about 1 mg to about 4 mg per 1.0 mg of Compound A.

In one embodiment, the invention relates to a pharmaceutical formulation comprising:

a) a pharmaceutically effective amount of Compound A having the structure

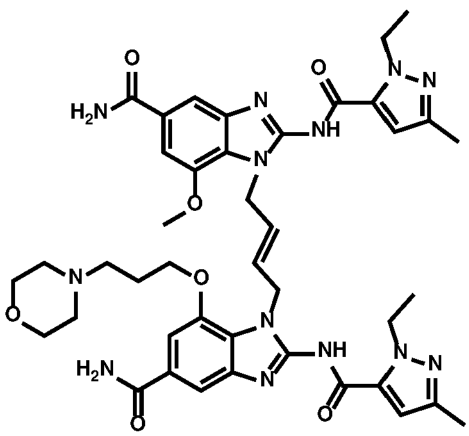

,

or a tautomer thereof,
or a pharmaceutically acceptable salt thereof;

b) a bulking agent or combination of bulking agents; and
c) aspartic acid in an amount from about 0.5 mg to about 10 mg per 1.0 mg of Compound A.

In one embodiment, the invention relates to a pharmaceutical formulation comprising:

a) a pharmaceutically effective amount of Compound A having the structure

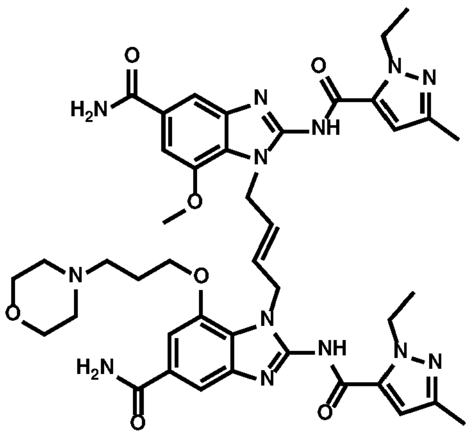,

or a tautomer thereof,
or a pharmaceutically acceptable salt thereof;

b) a bulking agent or combination of bulking agents; and
c) aspartic acid in an amount from about 0.5 mg to about 6 mg per 1.0 mg of Compound A.

In one embodiment, the invention relates to a pharmaceutical formulation comprising:

a) a pharmaceutically effective amount of Compound A having the structure

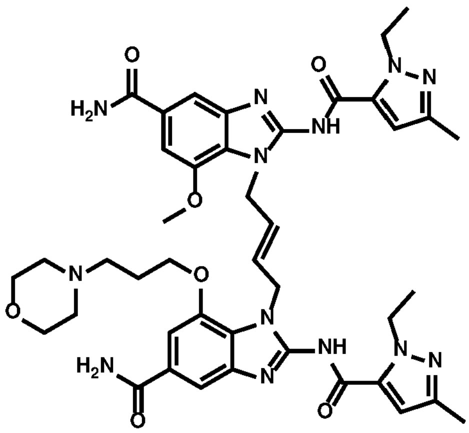,

or a tautomer thereof,
or a pharmaceutically acceptable salt thereof;

b) a bulking agent or combination of bulking agents; and
c) aspartic acid in an amount from about 1 mg to about 4 mg per 1.0 mg of Compound A.

In one embodiment, the invention relates to a pharmaceutical formulation comprising:

a) a pharmaceutically effective amount of Compound A having the structure

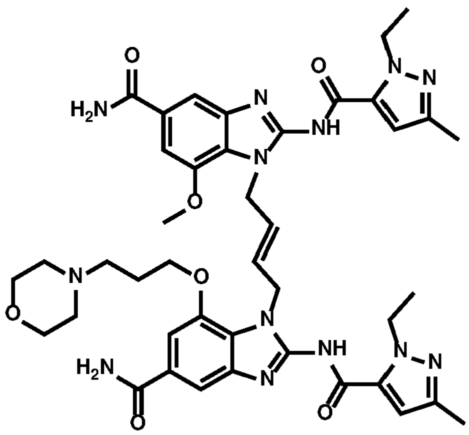

,

or a tautomer thereof,
or a pharmaceutically acceptable salt thereof;

b) a bulking agent or combination of bulking agents; and
c) aspartic acid in an amount from about 1.5 mg to about 3.5 mg per 1.0 mg of Compound A.

In one embodiment, the invention relates to a pharmaceutical formulation comprising:

a) a pharmaceutically effective amount of Compound A having the structure

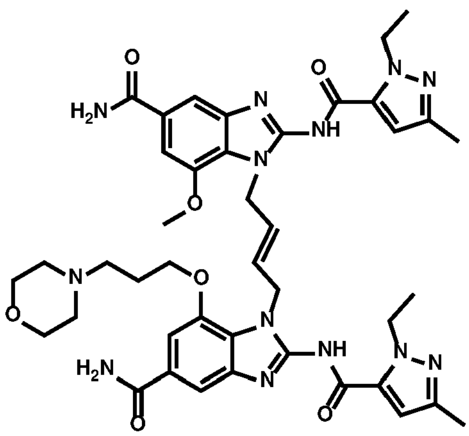

,

or a tautomer thereof,
or a pharmaceutically acceptable salt thereof;

b) a bulking agent or combination of bulking agents; and
c) aspartic acid in an amount from about 2 mg to about 3 mg per 1.0 mg of Compound A.

In one embodiment, the invention relates to a pharmaceutical formulation comprising:

a) from about 0.1 mg to about 4 mg of Compound A having the structure

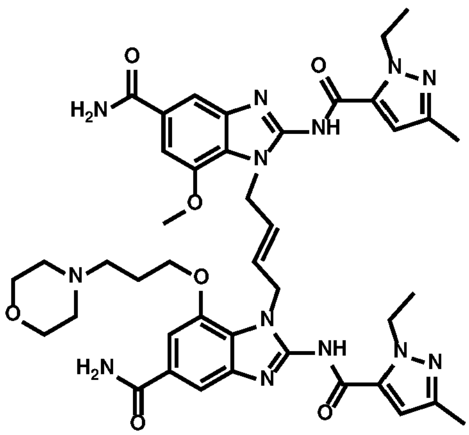

,

or a tautomer thereof,
or a pharmaceutically acceptable salt thereof;
b) a bulking agent or combination of bulking agents; and
c) aspartic acid in an amount from about 2 mg to about 3 mg per 1.0 mg of Compound A.

In one embodiment, the invention relates to a pharmaceutical formulation comprising:

a) from about 0.1 mg to about 4 mg of Compound A having the structure

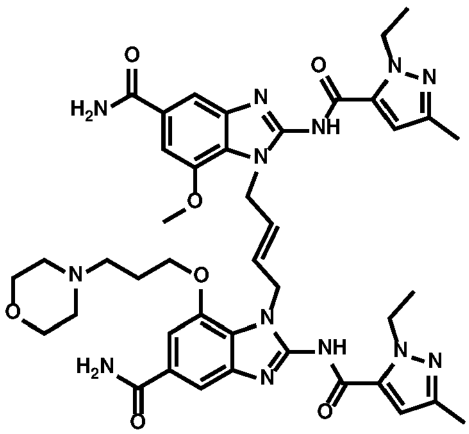

,

or a tautomer thereof,
or a pharmaceutically acceptable salt thereof;

b) a bulking agent or combination of bulking agents; and
c) a solubilizing agent selected from: aspartic acid, acetic acid, glutamic acid, methane sulfonic acid, lactic acid, and glucuronic acid, in a molar ratio of Compound A:solubilizing agent from about 1:1 to 1:25; and optionally
d) water.

In one embodiment, the invention relates to a pharmaceutical formulation comprising:

a) from about 0.5 mg to about 1.5 mg of Compound A having the structure

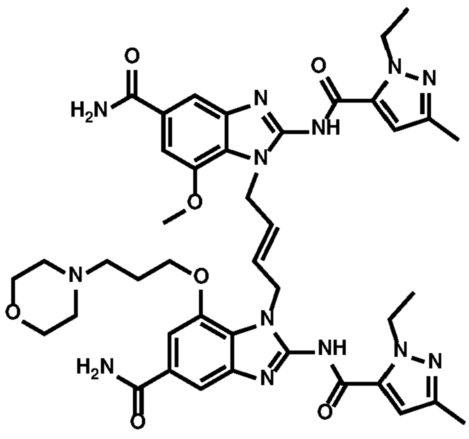,

or a tautomer thereof,
or a pharmaceutically acceptable salt thereof;

b) a bulking agent or combination of bulking agents; and
c) a solubilizing agent selected from: aspartic acid, acetic acid, glutamic acid, methane sulfonic acid, lactic acid, and glucuronic acid, in a molar ratio of Compound A:solubilizing agent from about 1:1 to 1:25; and optionally
d) water.

In one embodiment, the invention relates to a pharmaceutical formulation comprising:

a) a pharmaceutically effective amount of Compound A having the structure

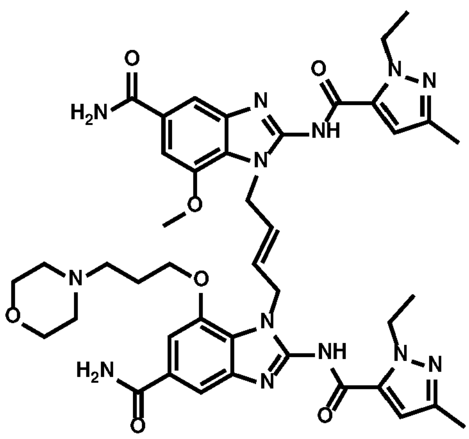,

or a tautomer thereof,
or a pharmaceutically acceptable salt thereof;

b) a bulking agent or combination of bulking agents; and
c) aspartic acid in an amount from about 2.66 mg per 1.0 mg of Compound A.

In one embodiment, the invention relates to a process to prepare a pharmaceutical formulation comprising the steps of:

1) combining into a mixture:

a) a pharmaceutically effective amount of Compound A having the structure

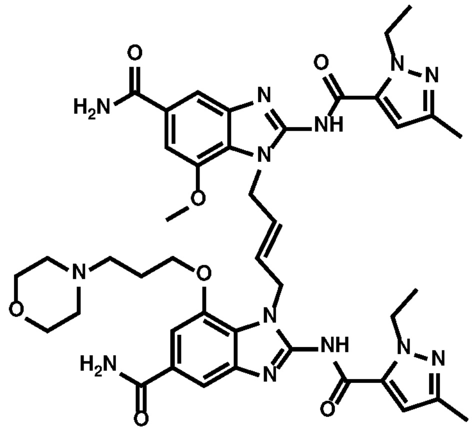

,

or a tautomer thereof,
or a pharmaceutically acceptable salt thereof;

b) a bulking agent or combination of bulking agents; and
c) a solubilizing agent selected from: aspartic acid, acetic acid, glutamic acid, methane sulfonic acid, lactic acid, and glucuronic acid;
d) water; and

2) optionally evaporating or lyophilizing the mixture to form a powder or cake.

In one embodiment, the invention relates to a process to prepare a pharmaceutical formulation comprising the steps of:

1) combining into a mixture:

a) a pharmaceutically effective amount of Compound A having the structure

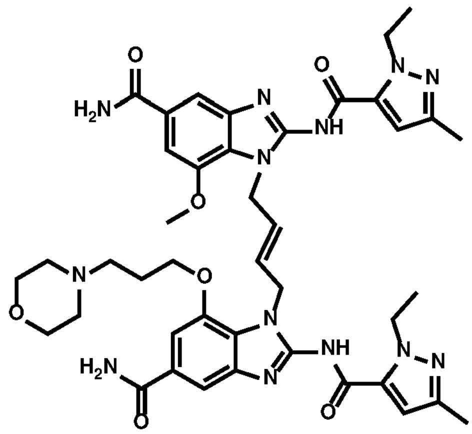

,

or a tautomer thereof,
or a pharmaceutically acceptable salt thereof;

b) a bulking agent or combination of bulking agents; and
c) a solubilizing agent selected from: aspartic acid, acetic acid, glutamic acid, methane sulfonic acid, lactic acid, and glucuronic acid, in an amount effective to provide a pH between about 2 and 4,

2) adding water to solubilize the mixture, and
3) evaporating the mixture to form a powder.

In one embodiment, the invention relates to a process to prepare a pharmaceutical formulation comprising the steps of:

1) combining into a mixture:

a) a pharmaceutically effective amount of Compound A having the structure

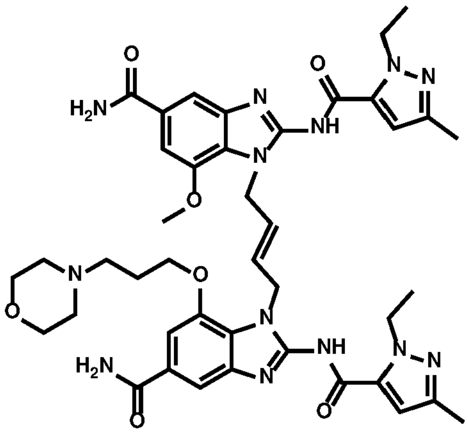

,

or a tautomer thereof,
or a pharmaceutically acceptable salt thereof;

b) a bulking agent or combination of bulking agents; and
c) a solubilizing agent selected from: aspartic acid, acetic acid, glutamic acid, methane sulfonic acid, lactic acid, and glucuronic acid, in an amount effective to provide a pH between about 2 and 4,

2) adding water to solubilize the mixture, and
3) lyophilizing the mixture to form a powder or cake.

In one embodiment, the invention relates to a process to prepare a pharmaceutical formulation comprising the steps of:

1) combining into a mixture:

a) a pharmaceutically effective amount of Compound A having the structure

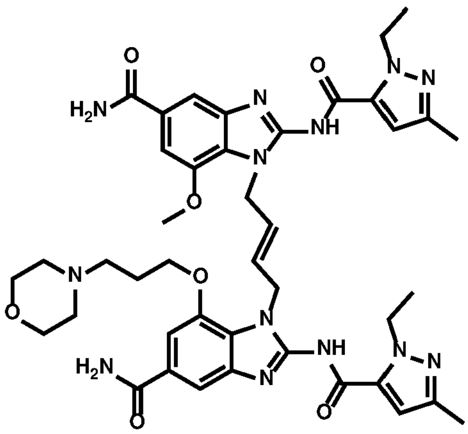

,

or a tautomer thereof,
or a pharmaceutically acceptable salt thereof;

b) a bulking agent or combination of bulking agents; and
c) a solubilizing agent selected from: aspartic acid, acetic acid, glutamic acid, methane sulfonic acid, lactic acid, and glucuronic acid, in an amount effective to provide a pH between about 2 and 4,

2) adding water to solubilize the mixture,
3) lyophilizing the mixture to form a powder or cake, and
4) optionally adding water, to form an injectable solution.

In one embodiment, the invention relates to a process to prepare a pharmaceutical formulation comprising the steps of:

1) combining into a mixture:

a) a pharmaceutically effective amount of Compound A having the structure

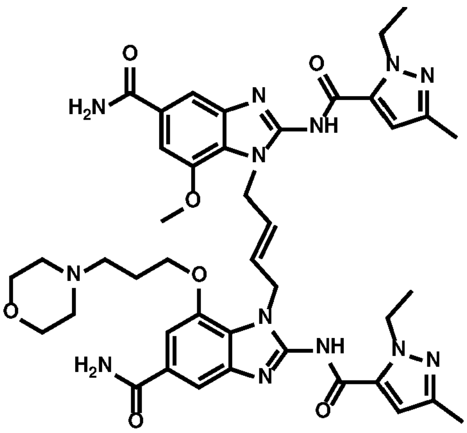

,

or a tautomer thereof,
or a pharmaceutically acceptable salt thereof;

b) a bulking agent or combination of bulking agents; and
c) a solubilizing agent selected from: aspartic acid, acetic acid, glutamic acid, methane sulfonic acid, lactic acid, and glucuronic acid, in an amount from about 0.3 mg to about 4 mg per 1.0 mg of Compound A,

2) adding water to solubilize the mixture, and
3) evaporating the mixture to form a powder, and
4) optionally adding water, to form an injectable solution.

In one embodiment, the invention relates to a process to prepare a pharmaceutical formulation comprising the steps of:

1) combining into a mixture:

a) a pharmaceutically effective amount of Compound A having the structure

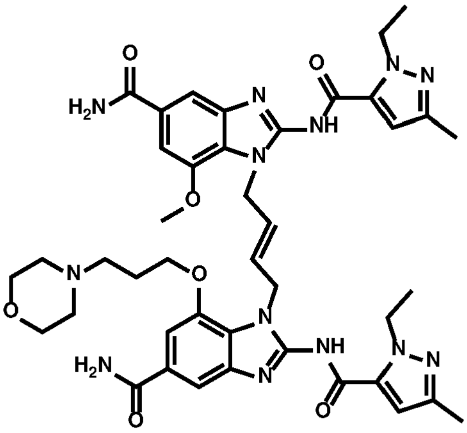

,

or a tautomer thereof,
or a pharmaceutically acceptable salt thereof;

b) a bulking agent or combination of bulking agents; and
c) a solubilizing agent selected from: aspartic acid, acetic acid, glutamic acid, methane sulfonic acid, lactic acid, and glucuronic acid, in a molar ratio of Compound A:solubilizing agent from about 1:10 to 1:25,

2) adding water to solubilize the mixture,
3) lyophilizing the mixture to form a powder or cake, and
4) optionally adding water, to form an injectable solution.

In one embodiment, the invention relates to a process to prepare a pharmaceutical formulation comprising the steps of:

1) combining into a mixture:

a) a pharmaceutically effective amount of Compound A having the structure

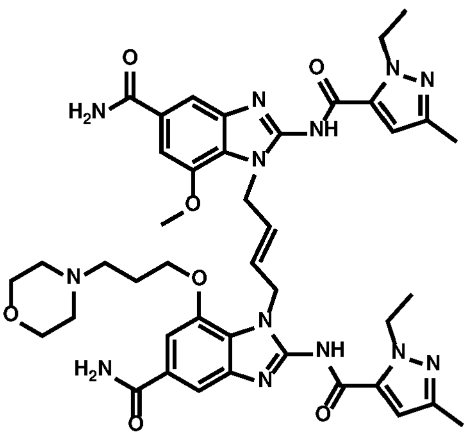

,

or a tautomer thereof,
or a pharmaceutically acceptable salt thereof;

b) a bulking agent or combination of bulking agents; and
c) aspartic acid in an amount from about 1 mg to about 4 mg per 1.0 mg of Compound A,

2) adding water to solubilize the mixture,
3) lyophilizing the mixture to form a powder or cake, and

4) optionally adding water, to form an injectable solution.

In one embodiment, the invention relates to a pharmaceutical formulation for intravenous administration to a patient in need thereof, comprising,

a) a pharmaceutically effective amount of Compound A having the structure

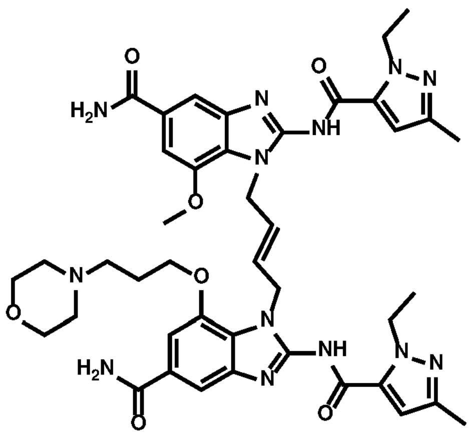

b) a pharmaceutically acceptable amount of a bulking agent or combination of bulking agents;
c) a pharmaceutically acceptable amount of a solubilizing agent selected from: aspartic acid, acetic acid, glutamic acid, and glucuronic acid, effective to provide a pharmaceutically acceptable pH; and optionally
d) water.

In one embodiment, the invention relates to a process for making a pharmaceutical formulation comprising Compound A having the structure:

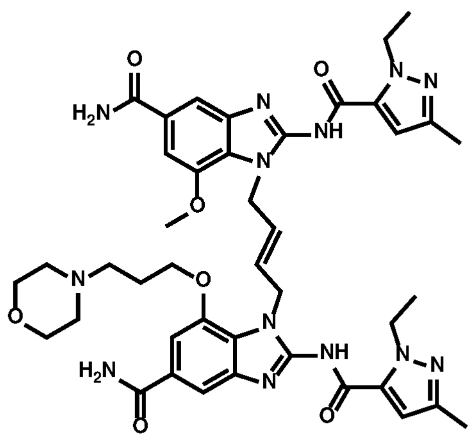

which comprises the steps of:

a) adding solubilizing agent selected from: aspartic acid, acetic acid, glutamic acid, and glucuronic acid, to water to form a solution;
b) adding Compound A to dissolve while maintaining pH between about 3.0 and 3.3;
c) dissolving a bulking agent or combination of bulking agents in the solution;
d) filtering the solution thus prepared;
e) optionally filling the filtered solution into a lyophilization vial and freezing at about -45 °C; and
f) optionally freeze drying the frozen solution.

In one embodiment, the invention relates to a method for treating a disease in which modulation of STING (Stimulator of Interferon Genes) is beneficial, in a patient in need thereof which comprises administering intravenously to said patient an effective amount of a formulation comprising from about 0.5 mg/ml to about 6 mg/ml of Compound A,

mannitol in an amount from about 20 mg/ml to about 200 mg/ml, aspartic acid in an amount of about 2.66 mg per 1.0 mg of Compound A, and water.

In one embodiment, the invention provides a pharmaceutical formulation of the present invention for use in the treatment of a disease in which modulation of STING is beneficial.

In one embodiment, the invention relates to a process for making a pharmaceutical formulation comprising Compound A having the structure:

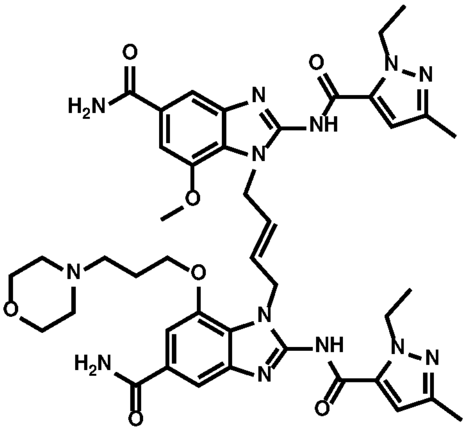

or a tautomer thereof,
or a pharmaceutically acceptable salt thereof;
which comprises the steps of:

a) forming a solution comprising

i) at least one solubilizing agent selected from: aspartic acid, acetic acid, glutamic acid, methane sulfonic acid, lactic acid, and glucuronic acid;
ii) Compound A, or a tautomer, or a pharmaceutically acceptable salt thereof, while maintaining pH between about 3.0 and 3.5;
iii) a bulking agent or combination of bulking agents; and
Iv) water;

b) optionally filtering the solution;
c) optionally filling the filtered solution into a lyophilization vial and freezing at about -45 °C; and
d) freeze drying the frozen solution or evaporating the non-frozen solution.

In one embodiment, the invention relates to a process for making a pharmaceutical formulation comprising Compound A having the structure:

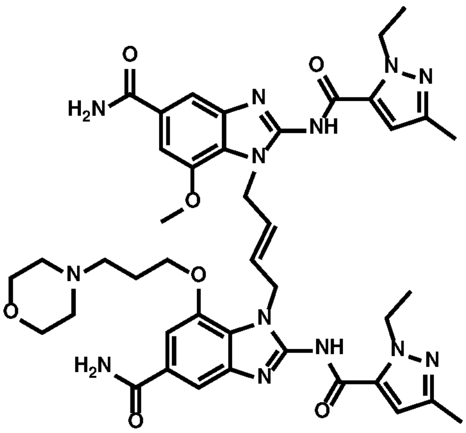

or a tautomer thereof,

or a pharmaceutically acceptable salt thereof;
which comprises the steps of:

a) forming a solution comprising

i) at least one solubilizing agent selected from: aspartic acid, acetic acid, glutamic acid, methane sulfonic acid, lactic acid, and glucuronic acid;
ii) Compound A, or a tautomer, or a pharmaceutically acceptable salt thereof, while maintaining pH between about 3.0 and 3.5;
iii) a bulking agent or combination of bulking agents; and Iv) water;

b) optionally filtering the solution;
c) optionally filling the filtered solution into a lyophilization vial and freezing at about -45 °C;
d) freeze drying the frozen solution or evaporating the non-frozen solution; and
e) optionally adding water, to form an injectable solution.

In one embodiment, the invention relates to a process for making a pharmaceutical formulation comprising Compound A having the structure:

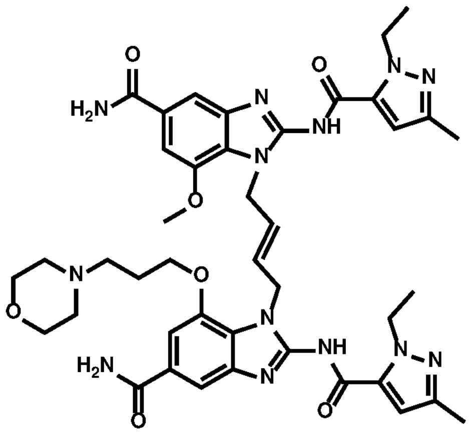

or a tautomer thereof,
or a pharmaceutically acceptable salt thereof;
which comprises the steps of:

a) forming a solution comprising

i) at least one solubilizing agent selected from: aspartic acid, acetic acid, glutamic acid, methane sulfonic acid, lactic acid, and glucuronic acid in a molar ratio of Compound A:solubilizing agent from about 1:1 to 1:125;
ii) from about 0.1 to about 4 mg of Compound A, or a tautomer, or a pharmaceutically acceptable salt thereof, while maintaining pH between about 3.0 and 3.5;
iii) a bulking agent or combination of bulking agents in an amount from about 20 mg to about 200 mg per 1.0 mg of Compound A; and
Iv) water;

b) optionally filtering the solution;
c) optionally filling the filtered solution into a lyophilization vial and freezing at about -45 °C;
d) freeze drying the frozen solution or evaporating the non-frozen solution; and
e) optionally adding water, to form an injectable solution.

In one embodiment, the invention relates to a process for making a pharmaceutical formulation comprising Compound A having the structure:

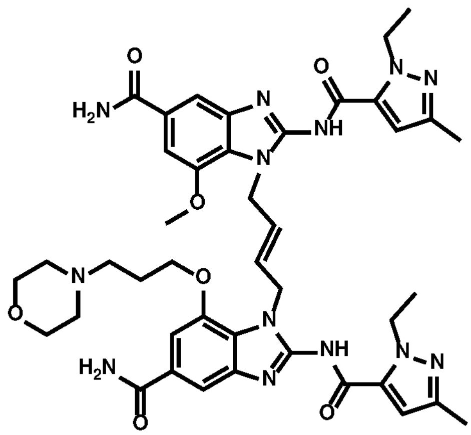

or a tautomer thereof,
or a pharmaceutically acceptable salt thereof;
which comprises the steps of:

a) forming a solution comprising

i) at least one solubilizing agent selected from: aspartic acid, acetic acid, glutamic acid, methane sulfonic acid, lactic acid, and glucuronic acid in a molar ratio of Compound A:solubilizing agent from about 1:1 to 1:100;
ii) from about 0.5 to about 1.5 mg of Compound A, or a tautomer, or a pharmaceutically acceptable salt thereof, while maintaining pH between about 3.0 and 3.5;
iii) a bulking agent or combination of bulking agents in an amount from about 20 mg to about 200 mg per 1.0 mg of Compound A; and
Iv) water;

b) optionally filtering the solution;
c) optionally filling the filtered solution into a lyophilization vial and freezing at about -45 °C;
d) freeze drying the frozen solution or evaporating the non-frozen solution; and
e) optionally adding water, to form an injectable solution.

In one embodiment, the invention relates to a process for making a pharmaceutical formulation comprising Compound A having the structure:

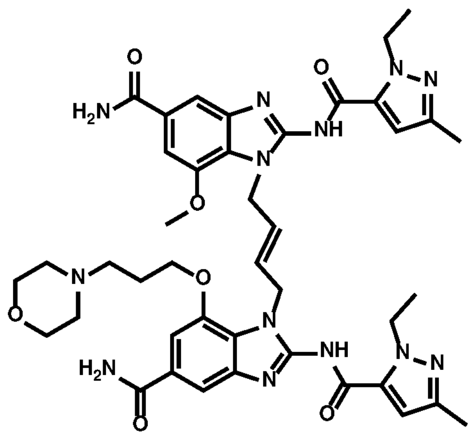

or a tautomer thereof,
or a pharmaceutically acceptable salt thereof;
which comprises the steps of:

a) forming a solution comprising

i) aspartic acid in an amount from about 2 mg to about 3 mg per 1.0 mg of Compound A;
ii) from about 0.5 to about 1.5 mg of Compound A, or a tautomer, or a pharmaceutically acceptable salt thereof, while maintaining pH between about 3.0 and 3.5;
iii) a bulking agent or combination of bulking agents in an amount from 80 mg to about 100 mg per 1.0 mg of Compound A; and
Iv) water;

b) optionally filtering the solution;
c) optionally filling the filtered solution into a lyophilization vial and freezing at about -45 °C;
d) freeze drying the frozen solution or evaporating the non-frozen solution; and
e) optionally adding water, to form an injectable solution.

Due to the poor oral bioavailability of Compound A, oral administration, for example by tablet, capsule, pellet, lozenge, or powder, was unobtainable. Consequently, a suitable formulation for parenteral administration was sought. To administer Compound A by parenteral administration, the compound must be made available in dissolved form in a suitable vehicle. A formulation concentration of ≥0.1 mg/mL, suitably ≥0.5 mg/mL, suitably ≥1.0 mg/mL, is desired to meet this need, which is a challenge, because of the low solubility of Compound A. Suitably, the concentration is within a range from 0.1 mg/mL to 4 mg/mL. The formulation composition of the present invention is designed to be soluble/bioavailable, satisfy pH and osmolality such that it can be injected "as is" via intravenous bolus or diluted further with a suitable medium, such as dextrose (D5W), for intravenous infusion, thereby solving the problem of formulating Compound A, a compound with poor bioavailability and low solubility, into an acceptable pharmaceutical dosage form.

One option in formulating Compound A for parenteral administration is to form a crystallized salt, hydrate or solvate of the compound that presents adequate solubility in formulation. Attempts to prepare a stable, crystalline form of Compound A as a salt, hydrate or solvate proved challenging. These alternative forms of Compound A were generally very hygroscopic, which is unsuitable for a pharmaceutical formulation as it tends to lead to dosage variation and increased degradation products.

An IV (intravenous) formulation was attempted. Formulations within the pH range of 4 to 9 are generally considered for IV administration. Formulations near the extremes of this pH range, especially the lower extreme, are of particular concern due to complications including: severe irritation at the injection site and thrombophlebitis. In order to minimize irritation upon administration, the pharmaceutical excipients, particularly the counterion and buffer capacity, of the formulation are critical. Formulations outside the physiological pH may have a tendency to precipitate in blood after injection due to decreased solubility at the normal human pH of 7.4 causing thrombophlebitis. (S. H. YALKOWSKY Journal of Pharmaceutical Sciences Vol. 87, No. 7, 787-796, July 1998). It is a purpose of this invention to provide an IV formulation of Compound A either directly or following dilution in a suitable medium, which avoids irritation at the injection site upon administration. It is also a purpose of this invention to prevent thrombophlebitis upon injection of a formulation of Compound A either directly or following dilution in a suitable medium. As indicated by Example 17, IV formulations of this invention have been found to be safe for IV bolus administration.

Surprisingly, it was found that the pHmax (pH of maximum solubility) for Compound A was dependent upon the counterion, where the concentration of the ionized form reached sufficient solubility to maintain a physically stable solution.

The formulations of the invention provide enhanced chemical stability and solubility to the pharmaceutical compositions. The use of selected solubilizing agents to prepare injectable doses and dry powder, suitably lyophilized, formulations of Compound A resulted in the generation of a stable and soluble formulation suitable for parenteral administration. A more stable formulation will result in an extended pharmaceutical shelf life which offers significant economic advantages.

It has been found that Compound A, particularly the freebase, is significantly more water soluble, and is more stable when in an aqueous formulation with a selected solubilizing agent or when reconstituted from lyophilized formulation prepared with a selected solubilizing agent, suitably the solubilizing agent is an organic acid. In a preferred embodiment, the solubilizing agent is selected from: aspartic acid, acetic acid, glutamic acid, methane sulfonic acid, lactic acid or glucuronic acid. It is understood that combinations of these acids are also useful in the invention. Suitably, the solubilizing agent is acetic acid. Suitably, the solubilizing agent is glutamic acid. Suitably, the solubilizing agent is methane sulfonic acid. Suitably, the solubilizing agent is lactic acid. Suitably, the solubilizing agent is glucuronic acid. Most preferably, the solubilizing agent is aspartic acid. Suitably the solubilizing agent is selected from: aspartic acid, acetic acid, glutamic acid, and glucuronic acid.

As used herein, the term aspartic acid includes all forms or aspartic acid including D-aspartic acid, L-aspartic acid, and combinations thereof, which are considered equivalents for use in current invention.

As used herein, unless otherwise defined, pH indications and ranges are all considered to be measured at room temperature (RT). Suitably RT is about 23 °C.

As used herein, Compound A, includes the compound in any form, i.e., any tautomeric form, any isomeric form, any salt or non-salt form (e.g., as a free acid or base form, or as a salt, particularly a pharmaceutically acceptable salt thereof) and any physical form thereof (e.g., including non-solid forms (e.g., liquid or semi-solid forms), and solid forms (e.g., amorphous or crystalline forms, specific polymorphic forms, solvate forms, including hydrate forms (e.g., mono-, di- and hemi- hydrates)), and mixtures of various forms.

It is understood that Compound A or a pharmaceutically acceptable salt thereof, or solvate (particularly, hydrates) thereof, may exist in crystalline forms, non-crystalline forms or a mixture thereof. The compound or salt, or solvates (particularly, hydrates) thereof, may also exhibit polymorphism (i.e. the capacity to occur in different crystalline forms). These different crystalline forms are typically known as "polymorphs." It is to be understood that the invention includes all polymorphs of Compound A, including any salts and/or solvates (particularly, hydrates) thereof, and mixtures thereof.

By utilizing a selected solubilizing agent, the product is more stable, contains less of unwanted degradation products, is highly soluble and extends the shelf life of the composition.

The invention is directed to a formulation comprising Compound A and excipients suitable for IV administration. It is understood that, unless otherwise indicated, the formulation can be an aqueous formulation that is ready to inject or a dry solid formulation, such as a powder formulation, suitably a lyophile formulation, that requires reconstitution prior to administration. When specifying the amounts of Compound A or excipients used in the invented formulations, the amount can be specified by weight, for example in mg, or by concentration, for example mg/mL, or by the effect the excipient has on the formulation, such as the amount needed to result in a specified pH or range. It is understood that, no matter how the amount is indicated, the amount is based on a concentration of mg/mL. For example, if the amount of aspartic acid is indicated as 2 mg/mL, that would equate to 2 mg of aspartic acid as a solid in a vial intended to be reconstituted to 1 mL or to an aqueous formulation that contains 2 mg of aspartic acid in every mL of water. If needed, the formulation can be subsequently diluted for infusion. The formulation of the invention includes an amount of selected solubilizing agent, suitably aspartic acid, suitably acetic acid, suitably glutamic acid, suitably glucuronic acid, suitably methane sulfonic acid, suitably lactic acid effective to provide a pH environment in the range of 2 to 4, suitably about 2.5 to about 3.8, suitably about 2.7 to about 3.6, suitably about 2.9 to about 3.5, suitably about 3.0 to about 3.5, suitably about 3, suitably about 3.1 suitably about 3.2, suitably about 3.3, suitably about 3.4, suitably about 3.5. Aspartic acid is particularly advantageous because it was unexpectedly found to solubilize Compound A in high concentrations, compared to other inorganic and organic acids. Depending on the amount relative to Compound A, aspartic acid solubilizes Compound A in the formulation up to about 6 mg/mL; suitably up to about 4 mg/mL, suitably from about 0.1 mg/mL to about 6 mg/mL; suitably from about 0.5 mg/mL to about 4 mg/mL; suitably from about 1 mg/mL to about 4 mg/mL; suitably from about 2 mg/mL to about 4 mg/mL; suitably about 2 mg/mL; suitably about 3 mg/mL.

Suitably Compound A is present in the dosage form in an amount from about 0.1 mg to about 6 mg; suitably from about 0.1 mg to about 4 mg; suitably from about 1 mg to about 5 mg; suitably from about 0.5 mg to about 2 mg; suitably from about 2 mg to about 4 mg; suitably about 0.1 mg; suitably about 0.2 mg; suitably about 0.3 mg; suitably about 0.4 mg; suitably about 0.5 mg; suitably about 0.6 mg; suitably about 0.7 mg; suitably about 0.8 mg; suitably about 0.9 mg; suitably about 1 mg; suitably about 1.25 mg; suitably about 1.5 mg; suitably about 2 mg; suitably about 3 mg; suitably about 4 mg.

One skilled in the art will understand the process of lyophilization and that this process results in the formation of a powder or cake. Both power and cake are considered equivalents in the art and as used herein.

Suitably Compound A is present in the formulation in an amount from about 0.5 mg to about 6 mg; suitably from about 1 mg to about 5 mg; suitably from about 2 mg to about 4 mg; suitably about 0.5 mg; suitably about 1 mg; suitably about 2 mg; suitably about 3 mg; suitably about 4 mg.

The amount of solubilizing agent, suitably L-aspartic acid, needed is dependent on the amount of Compound A in the formulation and is typically present in the range from about 0.5 mg/mL to about 4 mg/mL, suitably from about 1 mg/mL to about 3.5 mg/mL, suitably from about 1.5 mg/mL to about 3 mg/mL suitably, from about 2 mg/mL to about 3 mg/mL, suitably about 2.66 mg/mL.

The amount of solubilizing agent, suitably L-aspartic acid, needed is dependent on the amount of Compound A in the formulation and is typically present in the range from about 0.5 mg/mL to about 3 mg/mL, suitably from about 1 mg/mL to about 3 mg/mL.

Suitably the amount of solubilizing agent, suitably L-aspartic acid, in the formulation is about 2.66 mg per 1.0 mg of Compound A, suitably from about 0.1 mg to about 10 mg per 1.0 mg of Compound A, suitably from about 0.5 mg to about 10 mg per 1.0 mg of Compound A, suitably from about 0.5 mg to about 6 mg per 1.0 mg of Compound A, suitably from about 1 mg to about 4 mg per 1.0 mg of Compound A, suitably from about 1.5 mg to about 3.5 mg per 1.0 mg of Compound A, suitably from about 2 mg to about 3 mg per 1.0 mg of Compound A.

As used herein, when the amount of solubilizing agent, for example aspartic acid, is expressed in terms such as: effective to provide a specified pH, it is understood that amount of solubilizing agent intended is that amount that will provide the specified pH upon reconstitution, for example with 1 mL water, at room temperature. In one embodiment, the phrase "an amount effective to provide a specified pH" means an amount which provides the specified pH upon

adding water to a dry powder or lyophilized cake of the present invention in the amount of 0.5 mL, 1 mL, 2 mL, 3 mL, 4 mL, 5 mL, 6 mL, 7 mL, 8 mL, 9 mL, 10 mL, 15 mL, 20 mL, 30 mL, 40 mL, 50 mL, or 100 mL at room temperature.

Suitably, the formulations of the invention are provided in a sealed vial as a dry powder. In one embodiment, the dry powder is a lyophilized powder or cake that comprises Compound A; or an IV formulation comprising Compound A that is suitable for injection "as is" via intravenous bolus or for further dilution with a suitable medium, such as dextrose (D5W), for intravenous infusion.

As used herein bulking agents are excipients, e.g., excipient sugars, that are used to provide a suitable dry powder when formulated with Compound A. In one embodiment, the dry powder is a lyophilized powder or cake. Suitable bulking agents for use in the present invention include: mannitol, mannose, melibiose, octulose, fructose, lactose, sucrose, trehalose, sorbitol, glucose, galactose, glycine, dextrose, raffinose, ribose, xylitol, xylose, cyclodextrin, dextran, celluloses, povidone, PEG's (such as, for example, PEG 300, PEG 400, PEG 3350, PEG 6000, PEG 8000 and the like) polygalacturonic acid galacturonic acid, amino acids (including amino acid salts) such as lysine, arginine, glycine, and galactose), alone or in combination. In one embodiment, the dry powder provides for the dilution of the active ingredient throughout the powder and the final formulation. Suitable bulking agents useful in the invention consist essentially of: sucrose, lactose, mannitol, dextrose or combinations thereof. Suitably the bulking agent is mannitol, suitably D-mannitol. Suitable bulking agents useful in the invention include: sucrose, lactose, mannitol, dextrose or combinations thereof. Suitable bulking agents useful in the invention include: sucrose, lactose, mannitol or combinations thereof. The bulking agents are generally present in amounts from about 20 to about 200 mg/mL; suitably from about 40 to about 150 mg/mL; suitably from about 80 mg/mL to about 100 mg/mL, suitably about 48 mg/mL. The bulking agents are generally present in amounts from about 20 to about 200 mg per 1.0 mg of Compound A, suitably from about 40 to about 150 mg; suitably from about 80 mg to about 100 mg, suitably about 48 mg. Suitably the bulking agent is mannitol and the amount of mannitol in the formulation is about 48 mg per 1.0 mg of Compound A. In one embodiment, the indicated amount of bulking agent is sufficient to form a lyophilized powder or cake.

The compositions may also include other pharmaceutically acceptable diluents, excipients or carriers. The formulations are suitable for long-term storage in glass containers commonly used in the pharmaceutical industry, e.g., standard USP Type I borosilicate glass containers.

An aqueous injectable composition of Compound A (or drug as used herein) can be manufactured by bringing together excipients, including a solubilizing agent, for example aspartic acid, suitably L-aspartic acid, and drug in water at a suitable temperature. One aspect of this invention relates to controlling the ratio of drug:solubilizing agent, suitably drug:aspartic acid, so as to achieve the required drug solubility and pH in the formulation. This molar ratio of Compound A:solubilizing agent can generally range from about 1:1 to 1:125, suitably 1:1 to 1:100 suitably 1:1 to 1:30, suitably from about 1:10 to 1:30, suitably from about 1:10 to 1:25, suitably from about 1:15 to 1:25, preferably at a ratio of around 1:20. The aqueous injectable composition of Compound A can be manufactured by first dissolving the solubilizing agent, suitably aspartic acid, in water followed by dissolution of Compound A in the acid media at a suitable temperature. A bulking agent, suitably mannitol, is added to the above solution to adjust osmolality and the batch is QSed to required volume with water for injection. The solution may be aseptically filtered through sterile filters and aseptically filled into pre-washed, pre-sterilized/depyrogenated USP Type I clear glass vials for sealing. For manufacturing a lyophilized dosage form, the filled vials may then be lyophilized, stoppered with, for example, pre-washed, pre-sterilized 20 mm grey stoppers under nitrogen flush and sealed.

In one embodiment of the invention, the compositions of the invention are generally prepared as follows:

1. The selected solubilizing agent, suitably aspartic acid, suitably acetic acid, suitably glutamic acid, suitably glucuronic acid, suitably methane sulfonic acid, suitably lactic acid, is added to water to form a solution;
2. Compound A is added and dissolved with the pH maintained between about 2.0 and 4.0, such as between 3.0 and 3.5;
3. A bulking agent, suitably mannitol, is added to the solution;
4. The solution is filtered and filled into a lyophilization vial and frozen at about -45 °C;
5. The frozen formulation is freeze dried at about -20 °C, with a secondary drying between about 25 and 45 °C; and
6. Lyophilized vials are stoppered and stored at about 25 °C.

The prepared lyophilized formulation can be reconstituted at the time of administration with a suitable diluent, for example water, to obtain a finished concentration of Compound A, or a tautomer or a hydrate, or a pharmaceutically acceptable salt thereof, for example of about 1.0 mg/mL, for example about 2.0 mg/mL, for example of about 4 mg/mL, for example of about 0.5 mg/mL, for example of about 0.1 mg/mL, for example of about 0.2 mg/mL, for example of about 0.3 mg/mL, for example of about 0.4 mg/mL, for example of about 0.6 mg/mL, for example of about 0.7 mg/mL, for example of about 0.8 mg/mL , for example of about 0.9 mg/mL, for example of about 1.25 mg/mL, for example of about 1.5 mg/mL, which is suitable for intravenous administration to a patient in need of Compound A.

In one embodiment of the invention, the compositions of the invention are generally prepared as follows:

1. The selected solubilizing agent, suitably aspartic acid, suitably acetic acid, suitably glutamic acid, suitably glucuronic acid, suitably methane sulfonic acid, suitably lactic acid, is added to water to form a solution;
2. Compound A is added and dissolved with the pH maintained between about 2.0 and 4.0, such as between 3.0 and 3.5;
3. A bulking agent, suitably mannitol, is added to the solution;
4. The solution is filtered and transferred to a suitable container;
5. The formulation is evaporated to form a dry powder; and
6. The dry powder is entered into vials and stoppered.

The prepared dry powder formulation can be reconstituted at the time of administration with a suitable diluent, for example water, to obtain a finished concentration of Compound A, or a tautomer or a hydrate, or a pharmaceutically acceptable salt thereof, for example of about 1.0 mg/mL, for example about 2.0 mg/mL, for example of about 4 mg/mL, for example of about 0.5 mg/mL, for example of about 0.1 mg/mL, for example of about 0.2 mg/mL, for example of about 0.3 mg/mL, for example of about 0.4 mg/mL, for example of about 0.6 mg/mL, for example of about 0.7 mg/mL, for example of about 0.8 mg/mL , for example of about 0.9 mg/mL, for example of about 1.25 mg/mL, for example of about 1.5 mg/mL, which is suitable for intravenous administration to a patient in need of Compound A.

In one embodiment of the invention, the compositions of the invention are generally prepared as follows:

1. The selected solubilizing agent, suitably aspartic acid, suitably acetic acid, suitably glutamic acid, suitably glucuronic acid, suitably methane sulfonic acid, suitably lactic acid, is added to water to form a solution;
2. Compound A is added and dissolved with the pH maintained between about 2.0 and 4.0, such as between 3.0 and 3.5;
3. A bulking agent, suitably mannitol, is added to the solution; and
4. The solution is filtered and transferred to a suitable container for IV administration.

Parenteral administration constitutes a pertinent aspect of any injectable dosage form and there are several types of IV fluids available for this purpose. IV solutions are often prepared by mixing IV formulations with compatible IV fluids. The properties of IV solution should be such that sterility is not compromised and the drug remains physically and chemically stable in solution to cover a suitable in-use period following mixing of the formulation/reconstituted solution with the IV fluid until administration to the patient. Alternatively, the solution dosage form can be diluted in a suitable IV admixture medium, such as 5% dextrose, 5% mannitol for dilution to appropriate dose for administration. The Compound A lyophilized dosage form can be reconstituted with sterile water for injection to obtain a clear solution prior to IV administration using methods similar to that described for the solution dosage form.

The term "pharmaceutically effective amount" of an active ingredient shall mean the amount that will elicit the biological or medical response of a tissue, system or animal, suitably a human, that is being sought by a researcher or clinician.

Suitably, "patients" as used herein refers to human patients.

Compositions of the invention may be administered to patients for the treatment of diseases and disorders as disclosed in International Patent Application Publication Number WO 2017/175147 including: cancer, pre-cancerous syndromes, infectious diseases, Influenza, HIV, HCV, HPV and HBV infection; and in International Patent Application Publication Number WO 2019/069275, including inflammation, allergic and autoimmune diseases, skin warts, multiple sclerosis, and AIDS.

Suitably the compositions of the invention are administered to patients for the treatment of cancer selected from: brain (gliomas), glioblastomas, astrocytomas, glioblastoma multiforme, Bannayan-Zonana syndrome, Cowden disease, Lhermitte-Duclos disease, Wilm's tumor, Ewing's sarcoma, Rhabdomyosarcoma, ependymoma, medulloblastoma, head and neck, kidney, liver, melanoma, ovarian, pancreatic, adenocarcinoma, ductal adenocarcinoma, adenosquamous carcinoma, acinar cell carcinoma, glucagonoma, insulinoma, prostate, sarcoma, osteosarcoma, giant cell tumor of bone, thyroid, lymphoblastic T cell leukemia, chronic myelogenous leukemia, chronic lymphocytic leukemia, hairy-cell leukemia, acute lymphoblastic leukemia, acute myelogenous leukemia, chronic neutrophilic leukemia, acute lymphoblastic T cell leukemia, plasmacytoma, Immunoblastic large cell leukemia, mantle cell leukemia, multiple myeloma, megakaryoblastic leukemia, multiple myeloma, acute megakaryocytic leukemia, promyelocytic leukemia, erythroleukemia, malignant lymphoma, hodgkins lymphoma, non-hodgkins lymphoma, lymphoblastic T cell lymphoma, Burkitt's lymphoma, follicular lymphoma, neuroblastoma, bladder cancer, urothelial cancer, vulval cancer, cervical cancer, endometrial cancer, renal cancer, mesothelioma, esophageal cancer, salivary gland cancer, hepatocellular cancer, gastric cancer, nasopharangeal cancer, buccal cancer, cancer of the mouth, GIST (gastrointestinal stromal tumor) and testicular cancer.

Suitably the compositions of the invention are administered to patients for the treatment of cancer selected from: Non-Small Cell Lung Cancer (NSCLC), bladder, squamous cell carcinoma of the head and neck, cervical, endometrial, ovarian, pancreas, microsatellite stable colorectal cancer, microsatellite instable high colorectal cancer, gastric, esophageal endo, esophagogastric junction, esophageal, squamous, acute myeloid leukemia, and triple negative breast cancer.

Suitably the compositions of the invention are administered to patients for the treatment of pre-cancerous syndromes selected from: cervical intraepithelial neoplasia, monoclonal gammapathy of unknown significance (MGUS), myelodysplastic syndrome, aplastic anemia, cervical lesions, skin nevi (pre-melanoma), prostatic intraepithleial (intraductal) neoplasia (PIN), Ductal Carcinoma in situ (DCIS), colon polyps and severe hepatitis or cirrhosis.

The dosage regimen utilizing the compositions of the present invention is selected in accordance with a variety of factors, including age, weight, sex and medical condition of the patient; the severity of the condition to be treated; the route of administration; and the renal and hepatic function of the patient. An ordinarily skilled physician can readily determine and prescribe the effective amount of the drug required to prevent, counter, or arrest the progress of the condition.

Intravenously, preferred doses of Compound A will range from about 0.1 mg/mL to about 6 mg/mL; suitably 0.5 mg/mL to about 6 mg/mL; suitably 0.5 mg/mL to about 4 mg/mL; suitably 0.5 mg/mL to about 2 mg/mL; suitably 0.5 mg/mL to about 1.5 mg/mL; suitably 0.5 mg/mL to about 1 mg/mL; suitably from about 1 mg/mL to about 5 mg/mL; suitably from about 2 mg/mL to about 4 mg/ml; suitably 2 mg/mL; suitably 4 mg/mL. For example, a formulation of the invention should have from about 0.5 mg/mL to about 6 mg/mL of Compound A for administration to a 50 kg patient.

Intravenously, the most preferred doses of Compound A will range from about 0.1 mg/mL to about 4 mg/mL; suitably from about 0.1 mg/mL to about 3 mg/mL; suitably from about 2 mg/mL to about 4 mg/mL; suitably 2 mg/mL; suitably 1 mg/mL; suitably 0.1 mg/mL; suitably 0.2 mg/mL; suitably 0.3 mg/mL; suitably 0.4 mg/mL; suitably 0.5 mg/mL; suitably 0.6 mg/mL; suitably 0.7 mg/mL; suitably 0.8 mg/mL, suitably 0.9 mg/mL; suitably 1.25 mg/mL; suitably 1.5 mg/mL. For example, a formulation of the invention should have from about 0.1 mg/mL to about 4 mg/mL of Compound A for administration to a 50 kg patient.

Compound A, the active ingredient, can be prepared as described in International Patent Application Publication Number WO 2017/175147, Example 14.

As disclosed in International Patent Application Application No. WO 2017/175147, Compound A may exist in a tautomeric or an isomeric form, e.g., as

(E)-1-((E)-4-((E)-5-carbamoyl-2-((1-ethyl-3-methyl-1H-pyrazole-5-carbonyl)imino)-7-(3-morpholinopropoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide

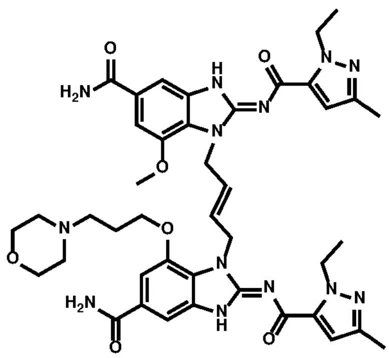

or (Z)-1-((E)-4-((Z)-5-carbamoyl-2-((1-ethyl-3-methyl-1H-pyrazole-5-carbonyl)imino)-7-(3-morpholinopropoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide

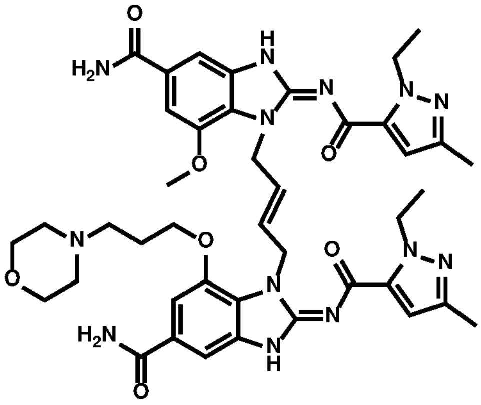

All such tautomeric and isomeric forms, including mixtures thereof, and pharmaceutically acceptable salts, solvates and hydrates thereof are included in Compound A.

As used herein, the term "formulation" and derivatives thereof, unless otherwise defined refers to dry powder, suitably a lyophilized powder or cake, comprising Compound A, a solution comprising Compound A reconstituted from a dry powder, suitably a lyophilized powder or cake, a solution ready for injection comprising Compound A, or a dry mixture of excipients and Compound A.

In another embodiment of the invention, there is provided a formulation and process for producing an aqueous injectable dosage form of Compound A. The formulation is listed below in Table A. The Compound A sterile solution dosage form composition presented in Table A is ready to inject since the pH and osmolality of formulation are controlled to avoid pain at the injection site.

**Table A**

| An injectable composition of Compound A | | |
|---|---|---|
| *Ingredient* | *Functionality* | *Quantities for 1 mL* |
| Compound A | Active | 1.0 mg |
| L-Aspartic acid | Solubilizer | 2.66 mg |
| Mannitol | Bulking agent | 48.0 mg |
| Water | Vehicle | QS to 1.0 mL |

In another embodiment of the invention, there is provided a composition of Compound A lyophile for injection and process for producing the composition. The composition is listed below in Table B.

**Table B**

| A composition of Compound A Lyophile for injection | | |
|---|---|---|
| *Ingredient* | *Functionality* | *Quantity per 10 cc vial* |
| Compound A | Active | 2.0 mg |
| L-Aspartic acid, USP/Ph.Eur | Solubilizer | 5.32 mg |
| Mannitol, USP/Ph.Eur | Bulking agent | 96.0 mg |
| Water for injection[1] | Vehicle | - |
| Nitrogen[2] | Headspace filler | - |
| 1 - Water for injection is removed during lyophilization.<br>2 - Nitrogen gas is used as inert gas for headspace. | | |

In another embodiment of the invention, the composition of Table B is compounded and filled into vials for lyophilization to obtain a lyophilized dosage form having suitable pharmaceutical properties such as stability and shelf life. In another embodiment of the invention, aspartic acid is used along with other acids, such as acetic acid, glucuronic

acid, lactic acid, methanesulfonic acid or an amino acid possessing at least one pKa ≤ 4, for example glutamic acid, to optimize the properties of the formulation such as stability and shelf life.

In another embodiment of the invention, there is provided a method of, or a formulation for, delivering Compound A by a parenteral route, including intravenous, subcutaneous, or intraperitoneal routes.

In another embodiment of the invention, there is provided a method of, or a formulation for, delivering Compound A by parenteral administration of a dosage form formulated as a solution, or a lyophile or a powder for reconstitution.

In another embodiment of the invention, there is provided a method of solubilizing, or a solubilized composition comprising, Compound A which utilizes the principle of pH-solubility enhancement of the free base of a drug with a suitable "solubilizing agent". A suitable solubilizing agent is one which enables solubility enhancement to the desired formulation of Compound A.

In another embodiment of the invention, there is provided a method to achieve solubility of, or a solubilized composition comprising, Compound A within a pH range of 2 to 4, suitably 3.0 to 3.5, for parenteral formulations. The desired solubility is based on dose requirements for the specific route of administration to achieve a physiological response upon parenteral administration.

In another embodiment of the invention, there is provided a method to achieve solubility of, or a solubilized composition comprising, Compound A prepared using an appropriate solubilizing agent such that the formulation pH lies within a pH range of 2 to 4, suitably 3.0 to 3.5, for parenteral formulations due to favorable pHmax.

In another embodiment of the invention, there is provided a method to formulate, or a formulated composition comprising, Compound A in the region of pHmax while maintaining the pH in the range of 2 to 4, suitably 3.0 to 3.5, suitably 3.0 to 3.2, suitably 3.2, suitably 3.5, using an excess of solubilizing agent.

In another embodiment of the invention, there is provided a method to formulate, or a formulated composition comprising, Compound A using an appropriate excess of the solubilizing agent as buffering agent to prevent disproportionation of free base, in addition to its primary role as counterion.

In another embodiment of the invention, there is provided a method to formulate, or a formulated composition comprising, Compound A which utilizes or utilizes the hydrogen bonding propensity of a zwitterionic solubilizing agent via inter-molecular interactions with the drug compound, wherein the zwitterionic solubilizing agent is preferably aspartic acid, suitably D-aspartic acid, suitably L-aspartic acid.

Contemplated equivalents:

Compound A, as utilized herein, may be in a free or unsalted form. It is contemplated that salts of Compound A, suitably pharmaceutically acceptable salts of Compound A, suitably non-hygroscopic pharmaceutically acceptable salts of Compound A, will perform similarly in the formulations of the invention. As such, salts of Compound A, suitably pharmaceutically acceptable salts of Compound A are contemplated equivalents in the formulations of the invention.

It is contemplated that the pH of an aqueous formulation of the invention could be adjusted between 2 and 4 by the addition of a minor amount of organic or inorganic acid other than aspartic acid, acetic acid, glutamic acid, lactic acid, or glucuronic acid, or a base, such as ammonium hydroxide, monoethanolamine, ethylene diamine, tromethamine, triethanolamine, suitably sodium hydroxide, without changing the utility of the formulation or the nature of the invention.

Without further elaboration, it is believed that one skilled in the art can, using the preceding description, utilize the present invention to its fullest extent. The following Examples are, therefore, to be construed as merely illustrative and not a limitation of the scope of the present invention in any way.

**Examples**

Compound A, as the free base, was tested with various solubilizing agents to see if a suitable agent could be identified to solubilize the compound in water.

**Examples 1 to 6 - Solubility screening (up to 5mg/mL at RT)**

Solubility of Compound A was conducted by adding a known quantity of drug to specific volume of aqueous media followed by 24 hour agitation at room temperature (RT). The results are presented below.

**Table 1**

| Compound A solubility up to 5 mg/mL in various aqueous media after stirring for 24 h at RT. | | | |
|---|---|---|---|
| **ACID media** | **pKa of acid** | **pH of acid media solution with Compound A** | **Drug Solubility*** (mg/mL) |
| 300 mM Phosphoric acid | 2.1 | 1.87 | 0.23 |
| 170 mM Acetic acid | 4.75 | 3.16 | 4.05 |
| 9.5 mM Lactic acid | 3.86 | 3.83 | 3.59 |
| 50 mM Citric acid | 3.13 | 2.30 | 1.32 |
| 115.7 mM Maleic acid | 1.9 | 1.52 | 0.15 |
| 72.8 mM Methane sulfonic acid | -2.6 | 1.27 | 3.85 |
| *Stirred for 24 h before centrifuging samples @8000 rpm for 15 min. Supernatant was analyzed after dilution by HPLC. | | | |

Table 1 shows the solubility of Compound A in different aqueous media with a targeted solubility up to 5 mg/mL. 300 mM phosphoric acid and 115.7 mM maleic acid media failed to solubilize 1 mg/mL of Compound A. 50 mM citric acid showed solubility <2 mg/mL. 9.5 mM lactic acid, 170 mM acetic acid and 72.8 mM methane sulfonic acid showed solubility >3.5 mg/mL.

**Examples 7 to 13** - **Solubility screening (up to 2 mg/mL at RT)**

**Table2**

| Compound A solubility up to 2 mq/mL in various aqueous media after stirring for | | | |
|---|---|---|---|
| **ACID media** | **pKa1 of acid** | **pH of acid media solution with Compound A** | **Drug Solubility*** (mg/mL) |
| 10 mM HCl | -7 | 1.98 | 0.36 |
| 10 mM Acetic acid | 4.8 | 3.33 | 1.45 |
| 10 mM Methane sulfonic acid | -1.2 | 1.99 | 1.78 |
| 25 mM Lactic acid | 3.86 | 2.57 | 1.83 |
| 25 mM Citric acid | 3.14 | 2.32 | 1.81 |
| 25 mM Aspartic acid | 1.9 | 2.95 | 1.80 |
| 25 mM Tartaric acid | 2.98 | 2.33 | 0.80 |
| *Stirred for 24 h before centrifuging samples @8000 rpm for 15 min. Supernatant was analyzed after dilution by HPLC. | | | |

Table 2 shows solubility of Compound A in different aqueous media with a targeted solubility up to 2 mg/mL. 10 mM hydrochloric acid and 25 mM tartaric acid media could not solubilize 1 mg/mL of Compound A. 10 mM acetic acid, 10 mM methane sulfonic acid, 25 mM lactic acid, 25 mM citric acid, 25 mM aspartic acid showed solubility >1 mg/mL. The above data indicates that, surprisingly, there is not a correlation between pKa of the acid solubilizing agent and Compound A solubility.

Acetic, citric and aspartic acids were explored further in Example 14.

**Example 14**

**Table 3**

| Compound A solubility in 20 mM acetic, citric and L-aspartic aqueous media as a function of pH at 22±1 °C with Compound A in excess amounts. | | | | | |
|---|---|---|---|---|---|
| **Citric acid media** | | **Acetic acid media** | | **L-aspartic acid media** | |
| **pH** | Solubility mg/mL | **pH** | Solubility mg/mL | **pH** | Solubility mg/mL |
| 2.47 | 0.4556 | 3.26 | 1.7587 | 3.05 | 5.2727 |
| 2.61 | 0.1947 | 3.58 | 1.0917 | 3.23 | 3.9676 |
| 2.74 | 0.0816 | 3.71 | 0.8103 | 3.76 | 0.4717 |
| 2.90 | 0.0415 | 3.91 | 0.3961 | 3.96 | 0.2269 |
| 3.40 | 0.0083 | 4.09 | 0.2686 | 4.18 | 0.0687 |
| 3.98 | 0.0054 | 4.64 | 0.0241 | 4.54 | 0.0167 |
| 4.94 | 0.004 | 4.85 | 0.0108 | 4.93 | 0.0050 |
| 5.91 | 0.0013 | 5.48 | 0.0029 | 6.14 | 0.0007 |
| 6.92 | 0.0005 | 6.42 | 0.0005 | 7.97 | 0.0005 |
| 8.82 | 0.0005 | 9.84 | 0.0005 | 8.87 | 0.0009 |
| *pH measured before addition of Compound A; pH adjustment achieved with addition of NaOH before addition of Compound A | | | | | |

As per the solubility data in Table 3, the rank ordering of counterion for solubility enhancement is: L-aspartic acid>acetic acid>citric acid. At pH 4.0, none of the counter ions showed solubility >0.5 mg/mL. L-aspartic acid offered the greatest solubility in the pH range of 3-3.5. Surprisingly, only acetic and L-aspartic acids showed adequate solubility, in the pH region 3.

**Example 15**

Aqueous solubility of Compound A with glutamic acid and glucuronic acid were explored in Example 15. The results are reported in Table 4

**Table 4**

| **Media** | **Adjusted pH** | **Solubility (mg/mL)[a]** |
|---|---|---|
| Glutamic acid (1 mol eq), aqueous | 4.1 | 4.5 |
| Glucuronic acid (1 mol eq), aqueous | 4.1 | 9.0 |
| [a] Solubility values are reported for filtered supernatants | | |

Summary of solubility screening from Examples 1 to 15.

Inorganic and organic solubilizing agents were screened for their ability to promote the solubility of Compound A in water. Counter ions derived from inorganic acids such as hydrochloric acid, and phosphoric acid did not offer sufficient solubility enhancement compared to the organic counter ions. Organic solubilizing agents examined were: methane sulfonic acid, acetic acid, citric acid, lactic acid, tartaric acid, maleic acid and aspartic acid. Additional organic solubilizing agents examined were: glutamic acid and glucuronic acid.

This data surprisingly demonstrated that the solubility was not only a function of pH but also depended on the nature of acid counterion. The equilibrium solubility data in Table 3 indicated that aspartic acid and acetic acid provided significantly better solubility at pH 3. Aspartic acid was found to provide the highest solubility at 4 mg/mL at pH <3.

**Example 16**

Compound A solubility was separately determined by controlling the mole ratio of L-aspartic acid relative to

drug in Example 16. The concentrations of drug and counterion were measured in supernatant to calculate Ksp (Solubility product) (Table 5).

**Table 5** pHmax determination of Compound A with L-aspartic acid (>110 hr stirring at RT)

| Drug Solubility (mg/mL) | Drug Solubility (M) | Counterion conc. (mM) | Ksp | $S_0$ (mg/mL) | $S_0$ (M) | pKa of drug | pH max* |
|---|---|---|---|---|---|---|---|
| 4.073 | 0.00479 | 8.45 | 4.05E-05 | 0.002 | 2.35E-06 | 7 | 3.57 |
| 3.946 | 0.00464 | 8.70 | 4.04E-05 | 0.002 | 2.35E-06 | 7 | 3.57 |
| 3.940 | 0.00464 | 9.18 | 4.26E-05 | 0.002 | 2.35E-06 | 7 | 3.56 |
| 4.144 | 0.00488 | 9.33 | 4.55E-05 | 0.002 | 2.35E-06 | 7 | 3.54 |
| *pHmax = pKa + log ($S_o/\sqrt{Ksp}$) | | | | | | | |

The Ksp measurements in Table 5 indicate that the pHmax for Compound A is (3.5-3.6) in L-aspartic acid media. A formulation between pH 3.0-3.5 would provide a safety margin for any pH shifts while maintaining the pH close to the generally acceptable pH range (4-9) for injectables.

**Example 17**

**Injection site reactions**

*In vivo* studies were conducted in cynomolgus monkeys administered IV doses of Compound A in a vehicle of 30 mM L-aspartic acid, 3.7% mannitol in Sterile Water for Injection, pH approximately 3.0. For these studies, Compound A was used in a non-stoichiometric hydrate form according to Table 6.

**Table 6**

| Type of Study | Route | Species (Strain) | Dose (mg/kg) | No.ISex/Group |
|---|---|---|---|---|
| **Repeat Dose**<br>3 weeks (3 doses) | IV (bolus) | Monkey (cynomolgus) | 0.1, 0.6<br>0.3, 1, 3 | 1M&1F |
| 4 weeks (5 doses) with 2 weeks off-dose period | IV (bolus) | Monkey (cynomolgus) | 0,03, 0.6, 1 | 3M & 3F (+2M & 2F for recovery at 0 & 1 mg/kg) |

Surprisingly, no Compound A-related injection site reactions were observed in monkeys following weekly IV bolus dosing of Compound A into the antecubital vein followed by a saline flush. These results support the conclusion that an IV formulation containing Compound A and aspartic acid with an approximate pH 3, was safe for human IV administration.

## Claims

**1.** A pharmaceutical formulation comprising,

a) a pharmaceutically effective amount of Compound A having the structure

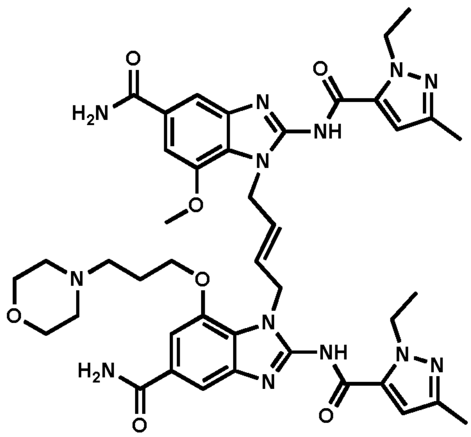

,

or a tautomer thereof,
or a pharmaceutically acceptable salt thereof;

b) a bulking agent or combination of bulking agents;
c) a solubilizing agent selected from: aspartic acid, acetic acid, glutamic acid, methane sulfonic acid, lactic acid, and glucuronic acid, and optionally
d) water.

**2.** The pharmaceutical formulation according to claim 1, wherein the amount of aspartic acid, acetic acid, glutamic acid, methane sulfonic acid, lactic acid or glucuronic acid, is effective to provide a pH of between about 2 and about 4.

**3.** The pharmaceutical formulation according to claim 1 or claim 2, wherein the molar ratio of Compound A: solubilizing agent is selected from about 1:1 to 1:125.

**4.** The pharmaceutical formulation according to any one of claims 1 to 3 wherein the solubilizing acid is in an amount from about 0.5 mg to about 10 mg per 1.0 mg of Compound A.

**5.** The pharmaceutical formulation according to any one of claims 1 to 4, wherein the solubilizing agent is aspartic acid.

**6.** The pharmaceutical formulation according to any one of claims 1 to 5, wherein the bulking agent or combination of bulking agents are in an amount effective to form a dry powder or lyophilized powder or cake.

**7.** The pharmaceutical formulation according to any one of claims 1 to 5, wherein the bulking agent or combination of bulking agents are in an amount from about 20 mg to about 200 mg per 1.0 mg of Compound A.

**8.** The pharmaceutical formulation according to any one of claims 1 to 5, wherein the bulking agent comprises: mannitol, mannose, melibiose, octulose, fructose, lactose, sucrose, trehalose, sorbitol, glucose, galactose, glycine, dextrose, raffinose, ribose, xylitol, xylose, cyclodextrin, dextran, celluloses, povidone, PEG 300, PEG 400, PEG 3350, PEG 6000, PEG 8000, polygalacturonic acid, galacturonic acid, lysine, arginine, glycine, or galactose, or a combination thereof.

**9.** The pharmaceutical formulation according to any one of claims 1 to 8, wherein the bulking agent consists essentially of mannitol.

**10.** The pharmaceutical formulation according to any one of claims 1 to 9, comprising from about 0.1 mg to about 4 mg of Compound A.

**11.** The pharmaceutical formulation according to any one of claims 1 to 10, where Compound A is in the form of a freebase.

**12.** The pharmaceutical formulation according to any one of claims 1 to 11, wherein the formulation comprises:

(i) 2 mg of compound A,

(ii) 96 mg mannitol, and
(iii) 5.32 mg of L-aspartic acid; or

wherein the formulation comprises:

(i) 1 mg of compound A,
(ii) 48 mg mannitol, and
(iii) 2.66 mg of L-aspartic acid; or

wherein the formulation comprises:

(i) 0.5 mg of compound A,
(ii) 48 mg mannitol, and
(iii) 1.3 mg of L-aspartic acid.

**13.** A process to prepare a pharmaceutical formulation comprising Compound A having the structure:

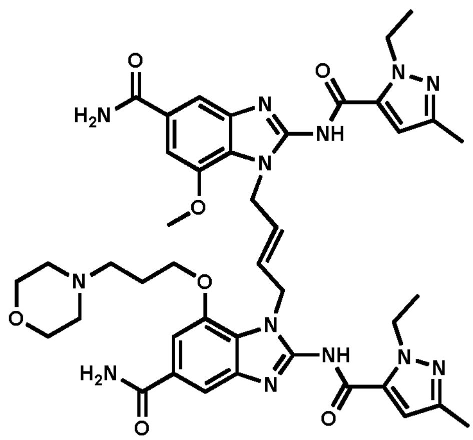

or a tautomer thereof,
or a pharmaceutically acceptable salt thereof;
which comprises the steps of:

a) forming a solution comprising

i) at least one solubilizing agent selected from: aspartic acid, acetic acid, glutamic acid, methane sulfonic acid, lactic acid, and glucuronic acid;
ii) Compound A, or a tautomer, or a pharmaceutically acceptable salt thereof, while maintaining pH between about 3.0 and 3.5;
iii) a bulking agent or combination of bulking agents; and
Iv) water;

b) optionally filtering the solution;
c) optionally filling the filtered solution into a lyophilization vial and freezing at about -45 °C; and
d) freeze drying the frozen solution or evaporating the non-frozen solution.

**14.** The process of claim 13 wherein:

a) the bulking agent is mannitol in an amount from about 20 mg to about 200 mg;
b) the solubilizing agent is L-aspartic acid in about 0.5 to 3.0 mg; and
c) Compound A, or a tautomer, or a pharmaceutically acceptable salt thereof, is in an amount selected from about 0.1 mg to about 2 mg.

**15.** A process to prepare a pharmaceutical formulation comprising the steps of:

1) combining into a mixture:

a) a pharmaceutically effective amount of Compound A having the structure

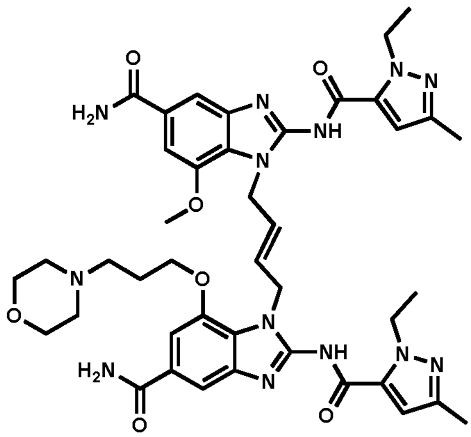

,

or a tautomer thereof,
or a pharmaceutically acceptable salt thereof;

b) a bulking agent or combination of bulking agents; and
c) a solubilizing agent selected from: aspartic acid, acetic acid, glutamic acid, methane sulfonic acid, lactic acid, and glucuronic acid;
d) water; and

2) optionally evaporating or lyophilizing the mixture to form a powder or cake.

**16.** A pharmaceutical formulation as defined in any one of claims 1 to 12 for use in the treatment of a disease in which modulation of STING (Stimulator of Interferon Genes) is beneficial selected from inflammation, allergic and autoimmune diseases, infectious diseases, hepatitis C virus (HCV), hepatitis B virus (HBV), influenza, skin warts, multiple sclerosis, human immunodeficiency virus (HIV) infection, AIDS, cancer, and pre-cancerous syndromes.

**17.** The pharmaceutical formulation of claim 16, wherein the use is in the treatment of cancer selected from: brain (gliomas), glioblastomas, astrocytomas, glioblastoma multiforme, Bannayan-Zonana syndrome, Cowden disease, Lhermitte-Duclos disease, Wilm's tumor, Ewing's sarcoma, Rhabdomyosarcoma, ependymoma, medulloblastoma, head and neck, kidney, liver, melanoma, ovarian, pancreatic, adenocarcinoma, ductal adenocarcinoma, adenosquamous carcinoma, acinar cell carcinoma, glucagonoma, insulinoma, prostate, sarcoma, osteosarcoma, giant cell tumor of bone, thyroid, lymphoblastic T cell leukemia, chronic myelogenous leukemia, chronic lymphocytic leukemia, hairy-cell leukemia, acute lymphoblastic leukemia, acute myelogenous leukemia, chronic neutrophilic leukemia, acute lymphoblastic T cell leukemia, plasmacytoma, Immunoblastic large cell leukemia, mantle cell leukemia, multiple myeloma, megakaryoblastic leukemia, multiple myeloma, acute megakaryocytic leukemia, promyelocytic leukemia, erythroleukemia, malignant lymphoma, hodgkins lymphoma, non-hodgkins lymphoma, lymphoblastic T cell lymphoma, Burkitt's lymphoma, follicular lymphoma, neuroblastoma, bladder cancer, urothelial cancer, vulval cancer, cervical cancer, endometrial cancer, renal cancer, mesothelioma, esophageal cancer, salivary gland cancer, hepatocellular cancer, gastric cancer, nasopharangeal cancer, buccal cancer, cancer of the mouth, GIST (gastrointestinal stromal tumor) and testicular cancer.

**18.** The pharmaceutical formulation of claim 16, wherein the use is in the treatment of cancer selected from: Non-Small Cell Lung Cancer (NSCLC), bladder, squamous cell carcinoma of the head and neck, cervical, endometrial, ovarian, pancreas, microsatellite stable colorectal cancer, microsatellite instable high colorectal cancer, gastric, esophageal endo, esophagogastric junction, esophageal, squamous, acute myeloid leukemia, and triple negative breast cancer.

**19.** The pharmaceutical formulation of claim 16, wherein the use is in the treatment of pre-cancerous syndromes selected from: cervical intraepithelial neoplasia, monoclonal gammapathy of unknown significance (MGUS), myelodysplastic syndrome, aplastic anemia, cervical lesions, skin nevi (pre-melanoma), prostatic intraepithleial (intraductal) neoplasia (PIN), Ductal Carcinoma in situ (DCIS), colon polyps and severe hepatitis or cirrhosis.

## Patentansprüche

1. Pharmazeutische Formulierung, umfassend

   a) eine pharmazeutisch wirksame Menge von Verbindung A mit der Struktur

   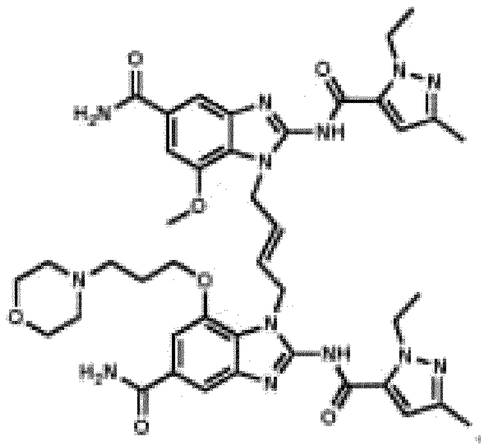

   oder ein Tautomer hiervon,
   oder ein pharmazeutisch verträgliches Salz hiervon;

   b) einen Füllstoff oder eine Kombination von Füllstoffen;
   c) einen Lösungsvermittler, ausgewählt aus:
   Asparaginsäure, Essigsäure, Glutaminsäure, Methansulfonsäure, Milchsäure und Glucuronsäure, und gegebenenfalls
   d) Wasser.

2. Pharmazeutische Formulierung nach Anspruch 1, wobei die Menge an Asparaginsäure, Essigsäure, Glutaminsäure, Methansulfonsäure, Milchsäure oder Glucuronsäure wirksam ist, um einen pH-Wert zwischen etwa 2 und etwa 4 bereitzustellen.

3. Pharmazeutische Formulierung nach Anspruch 1 oder 2, wobei das Molverhältnis von Verbindung A: Lösungsvermittler aus etwa 1:1 bis 1:125 ausgewählt ist.

4. Pharmazeutische Formulierung nach einem der Ansprüche 1 bis 3, wobei die solubilisierende Säure in einer Menge von etwa 0,5 mg bis etwa 10 mg pro 1,0 mg Verbindung A vorliegt.

5. Pharmazeutische Formulierung nach einem der Ansprüche 1 bis 4, wobei der Lösungsvermittler Asparaginsäure ist.

6. Pharmazeutische Formulierung nach einem der Ansprüche 1 bis 5, wobei der Füllstoff oder die Kombination von Füllstoffen in einer Menge vorliegt, die wirksam ist, um ein trockenes Pulver oder lyophilisiertes Pulver oder Kuchen zu bilden.

7. Pharmazeutische Formulierung nach einem der Ansprüche 1 bis 5, wobei der Füllstoff oder die Kombination von Füllstoffen in einer Menge von etwa 20 mg bis etwa 200 mg pro 1,0 mg Verbindung A vorliegt.

8. Pharmazeutische Formulierung nach einem der Ansprüche 1 bis 5, wobei der Füllstoff umfasst: Mannitol, Mannose, Melibiose, Oktulose, Fruktose, Laktose, Saccharose, Trehalose, Sorbitol, Glukose, Galactose, Glycin, Dextrose, Raffinose, Ribose, Xylitol, Xylose, Cyclodextrin, Dextran, Cellulosen, Povidon, PEG 300, PEG 400, PEG 3350, PEG 6000, PEG 8000, Polygalacturonsäure, Galacturonsäure, Lysin, Arginin, Glycin oder Galactose, oder eine Kombination hiervon.

**9.** Pharmazeutische Formulierung nach einem der Ansprüche 1 bis 8, wobei der Füllstoff im Wesentlichen aus Mannitol besteht.

**10.** Pharmazeutische Formulierung nach einem der Ansprüche 1 bis 9, umfassend etwa 0,1 mg bis etwa 4 mg Verbindung A.

**11.** Pharmazeutische Formulierung nach einem der Ansprüche 1 bis 10, wobei Verbindung A in Form einer freien Base vorliegt.

**12.** Pharmazeutische Formulierung nach einem der Ansprüche 1 bis 11, wobei die Formulierung umfasst:

(i) 2 mg Verbindung A,
(ii) 96 mg Mannitol und
(iii) 5,32 mg L-Asparaginsäure; oder

wobei die Formulierung umfasst:

(i) 1 mg Verbindung A,
(ii) 48 mg Mannitol und
(iii) 2,66 mg L-Asparaginsäure; oder

wobei die Formulierung umfasst:

(i) 0,5 mg Verbindung A,
(ii) 48 mg Mannitol und
(iii) 1,3 mg L-Asparaginsäure.

**13.** Verfahren zur Herstellung einer pharmazeutischen Formulierung, umfassend Verbindung A mit der Struktur:

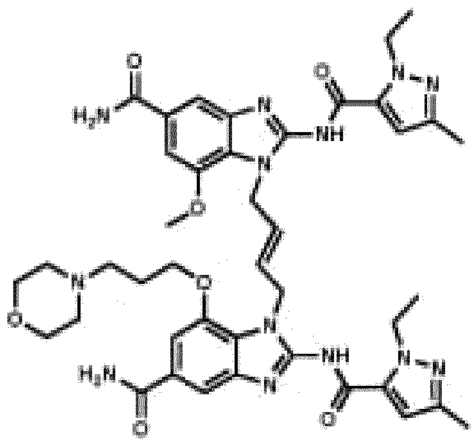

oder ein Tautomer hiervon,
oder ein pharmazeutisch verträgliches Salz hiervon;
welches die folgenden Schritte umfasst:

a) Bilden einer Lösung, umfassend

i) mindestens einen Lösungsvermittler, ausgewählt aus: Asparaginsäure, Essigsäure, Glutaminsäure, Methansulfonsäure, Milchsäure und Glucuronsäure;
ii) Verbindung A oder ein Tautomer oder ein pharmazeutisches verträgliches Salz hiervon, wobei der pH-Wert zwischen etwa 3,0 und 3,5 gehalten wird;
iii) einen Füllstoff oder eine Kombination von Füllstoffen; und
iv) Wasser;

b) gegebenenfalls Filtrieren der Lösung;
c) gegebenenfalls Abfüllen der gefilterten Lösung in ein Lyophilisierungsfläschchen und Einfrieren bei etwa -45°C; und
d) Gefriertrocknung der gefrorenen Lösung oder Eindampfen der nicht-gefrorenen Lösung.

**14.** Verfahren nach Anspruch 13, wobei:

a) der Füllstoff Mannitol in einer Menge von etwa 20 mg bis etwa 200 mg vorliegt;
b) der Lösungsvermittler L-Asparaginsäure in einer Menge von etwa 0,5 bis 3,0 mg ist; und
c) Verbindung A oder ein Tautomer oder ein pharmazeutisch verträgliches Salz hiervon in einer Menge vorliegt, die von etwa 0,1 mg bis etwa 2 mg ausgewählt ist.

**15.** Verfahren zur Herstellung einer pharmazeutischen Formulierung, umfassend die folgenden Schritte:

1) Folgendes zu einem Gemisch zu kombinieren:

a) eine pharmazeutisch wirksame Menge von Verbindung A mit der Struktur

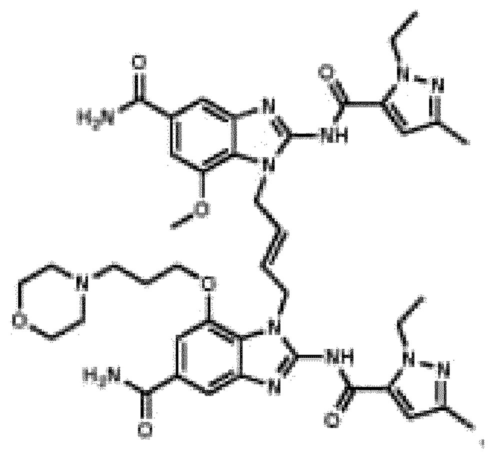

oder ein Tautomer hiervon,
oder ein pharmazeutisch verträgliches Salz hiervon;

b) ein Füllstoff oder eine Kombination von Füllstoffen; und
c) ein Lösungsvermittler, ausgewählt aus: Asparaginsäure, Essigsäure, Glutaminsäure, Methansulfonsäure, Milchsäure und Glucuronsäure;
d) Wasser; und

2) gegebenenfalls Eindampfen oder Lyophilisieren des Gemisches, um ein Pulver oder einen Kuchen zu bilden.

**16.** Pharmazeutische Formulierung nach einem der Ansprüche 1 bis 12 zur Anwendung bei der Behandlung einer Krankheit, bei der die Modulation von STING (Stimulator von Interferon-Genen) von Vorteil ist, ausgewählt aus Entzündungen, allergischen und Autoimmunerkrankungen, Infektionskrankheiten, Hepatitis C-Virus (HCV), Hepatitis B-Virus (HBV), Influenza, Hautwarzen, Multiple Sklerose, Infektion mit dem humanen Immundefizienzvirus (HIV), AIDS, Krebs und präkanzerösen Syndromen.

**17.** Pharmazeutische Formulierung nach Anspruch 16, wobei die Anwendung bei der Behandlung von Krebs, ausgewählt aus: Gehirn (Gliome), Glioblastome, Astrozytome, Glioblastoma multiforme, Bannayan-Zonana-Syndrom, Cowden-Krankheit, Lhermitte-Duclos-Krankheit, Wilm-Tumor, Ewing-Sarkom, Rhabdomyosarkom , Ependymom, Medulloblastom, Kopf und Hals, Niere, Leber, Melanom, Eierstock, Bauchspeicheldrüse, Adenokarzinom, duktales Adenokarzinom, adenosquamöses Karzinom, Azinuszellkarzinom, Glucagonom, Insulinom, Prostata, Sarkom, Osteosarkom, Riesenzelltumor der Knochen, Schilddrüse, Lymphoblast T-Zell-Leukämie, chronische myeloische Leukämie, chronische lymphatische Leukämie, Haarzellen-Leukämie, akute lymphatische Leukämie, akute myeloische Leukämie, chronische neutrophile Leukämie, akute lymphoblastische T-Zell-Leukämie, Plasmozytom, immunoblastische

großzellige Leukämie, Mantelzell-Leukämie, multiples Myelom, megakaryoblastische Leukämie, multiples Myelom, akute megakaryozytäre Leukämie, Promyelozytäre Leukämie, Erythroleukämie, malignes Lymphom, Hodgkin-Lymphom, Nicht-Hodgkin-Lymphom, lymphoblastisches T-Zell-Lymphom, Burkitt-Lymphom, follikuläres Lymphom, Neuroblastom, Blasenkrebs, Urothelkrebs, Vulvakrebs, Gebärmutterhalskrebs, Endometriumkrebs, Nierenkrebs, Mesotheliom, Speiseröhrenkrebs, Speicheldrüsenkrebs, Leberzellkrebs, Magenkrebs, Nasopharynxkrebs, bukkaler Krebs, Mundkrebs, GIST (gastrointestinaler Stromatumor) und Hodenkrebs, erfolgt.

**18.** Pharmazeutische Formulierung nach Anspruch 16, wobei die Anwendung bei der Behandlung von Krebs, ausgewählt aus: nicht-kleinzelligem Lungenkrebs (NSCLC), Blasenkrebs, Plattenepithelkarzinom des Kopfes und Halses, Gebärmutterhals-, Endometrium-, Eierstock-, Bauchspeicheldrüsenkrebs, Mikrosatelliten stabiler Dickdarmkrebs, Mikrosatelliten instabiler Dickdarmkrebs, Magenkrebs, ösophagealer endo-Krebs, Krebs des gastroösophagealen Übergangs, Speiseröhrenkrebs, Plattenepithelkarzinom, akute myeloische Leukämie und dreifach negativer Brustkrebs, erfolgt.

**19.** Pharmazeutische Formulierung nach Anspruch 16, wobei die Anwendung bei der Behandlung von präkanzerösen Syndromen, ausgewählt aus: zervikaler intraepithelialer Neoplasie, monoklonaler Gammapathie unbekannter Signifikanz (MGUS), myelodysplastischem Syndrom, aplastischer Anämie, zervikalen Läsionen, Hautnävi (Prämelanom), intraepitheliale (intraduktale) Neoplasie der Prostata (PIN), duktalem Carcinoma in situ (DCIS), Dickdarmpolypen und schwerer Hepatitis oder Zirrhose, erfolgt.

## Revendications

**1.** Formulation pharmaceutique comprenant :

a) une quantité pharmaceutiquement efficace du Composé A ayant la structure

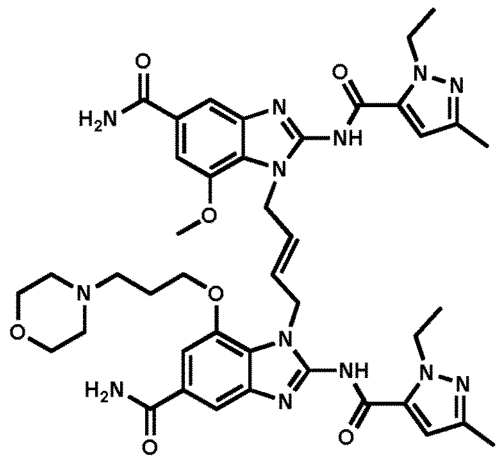

,

ou un tautomère de celui-ci,
ou un sel pharmaceutiquement acceptable de celui-ci ;

b) un agent de charge ou une combinaison d'agents de charge ;
c) un agent solubilisant choisi parmi : l'acide aspartique, l'acide acétique, l'acide glutamique, l'acide méthane sulfonique, l'acide lactique et l'acide glucuronique, et optionnellement
d) de l'eau.

**2.** Formulation pharmaceutique selon la revendication 1, dans laquelle la quantité d'acide aspartique, d'acide acétique, d'acide glutamique, d'acide méthane sulfonique, d'acide lactique ou d'acide glucuronique est efficace pour donner un pH compris entre environ 2 et environ 4.

**3.** Formulation pharmaceutique selon la revendication 1 ou la revendication 2, dans laquelle le rapport molaire du Composé A :agent solubilisant est choisi dans une valeur d'environ 1:1 à 1:125.

**4.** Formulation pharmaceutique selon l'une quelconque des revendications 1 à 3, dans laquelle l'acide solubilisant est présent en une quantité d'environ 0,5 mg à environ 10 mg pour 1,0 mg de Composé A.

**5.** Formulation pharmaceutique selon l'une quelconque des revendications 1 à 4, dans laquelle l'agent solubilisant est de l'acide aspartique.

**6.** Formulation pharmaceutique selon l'une quelconque des revendications 1 à 5, dans laquelle l'agent de charge ou la combinaison d'agents de charge est présent en une quantité efficace pour former une poudre sèche ou une poudre ou un gâteau lyophilisé.

**7.** Formulation pharmaceutique selon l'une quelconque des revendications 1 à 5, dans laquelle l'agent de charge ou la combinaison d'agents de charge est présent en une quantité d'environ 20 mg à environ 200 mg pour 1,0 mg de Composé A.

**8.** Formulation pharmaceutique selon l'une quelconque des revendications 1 à 5, dans laquelle l'agent de charge comprend : mannitol, mannose, mélibiose, octulose, fructose, lactose, saccharose, tréhalose, sorbitol, glucose, galactose, glycine, dextrose, raffinose, ribose, xylitol, xylose, cyclodextrine, dextrane, celluloses, povidone, PEG 300, PEG 400, PEG 3350, PEG 6000, PEG 8000, acide polygalacturonique, acide galacturonique, lysine, arginine, glycine ou galactose, ou une combinaison de ceux-ci.

**9.** Formulation pharmaceutique selon l'une quelconque des revendications 1 à 8, dans laquelle l'agent de charge consiste essentiellement en du mannitol.

**10.** Formulation pharmaceutique selon l'une quelconque des revendications 1 à 9, comprenant d'environ 0,1 mg à environ 4 mg de Composé A.

**11.** Formulation pharmaceutique selon l'une quelconque des revendications 1 à 10, où le Composé A se présente sous la forme d'une base libre.

**12.** Formulation pharmaceutique selon l'une quelconque des revendications 1 à 11, dans laquelle la formulation comprend :

(i) 2 mg de composé A,
(ii) 96 mg de mannitol, et
(iii) 5,32 mg d'acide L-aspartique ; ou

dans laquelle la formulation comprend :

(i) 1 mg de composé A,
(ii) 48 mg de mannitol, et
(iii) 2,66 mg d'acide L-aspartique ; ou

dans laquelle la formulation comprend :

(i) 0,5 mg de composé A,
(ii) 48 mg de mannitol, et
(iii) 1,3 mg d'acide L-aspartique.

**13.** Procédé de préparation d'une formulation pharmaceutique comprenant le Composé A ayant la structure :

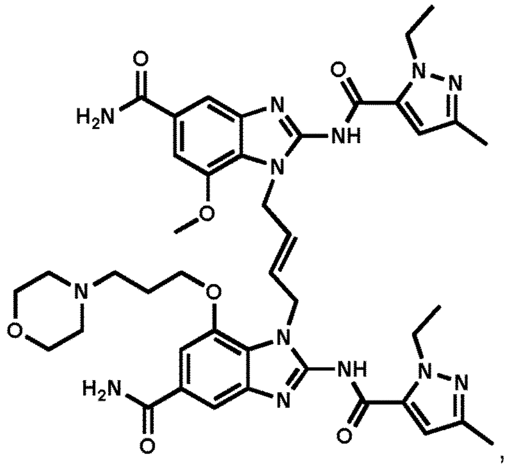

ou un tautomère de celui-ci,
ou un sel pharmaceutiquement acceptable de celui-ci ;
qui comprend les étapes de :

a) formation d'une solution comprenant

i) au moins un agent solubilisant choisi parmi : l'acide aspartique, l'acide acétique, l'acide glutamique, l'acide méthane sulfonique, l'acide lactique et l'acide glucuronique ;
ii) le Composé A ou un tautomère, ou un sel pharmaceutiquement acceptable de celui-ci, en maintenant le pH entre environ 3,0 et 3,5 ;
iii) un agent de charge ou une combinaison d'agents de charge ; et
iv) de l'eau ;

b) optionnellement, filtration de la solution ;
c) optionnellement remplissage de la solution filtrée dans un flacon de lyophilisation et congélation à environ -45 °C ; et
d) lyophilisation de la solution congelée ou évaporation de la solution non congelée.

**14.** Procédé selon la revendication 13, dans lequel :

a) l'agent de charge est le mannitol, en une quantité d'environ 20 mg à environ 200 mg ;
b) l'agent solubilisant est l'acide L-aspartique à raison d'environ 0,5 à 3,0 mg ; et
c) le Composé A ou un tautomère, ou un sel pharmaceutiquement acceptable de celui-ci, est présent en une quantité choisie d'environ 0,1 mg à environ 2 mg.

**15.** Procédé de préparation d'une formulation pharmaceutique comprenant les étapes de :

1) combinaison en un mélange :

a) d'une quantité pharmaceutiquement efficace du Composé A ayant la structure :

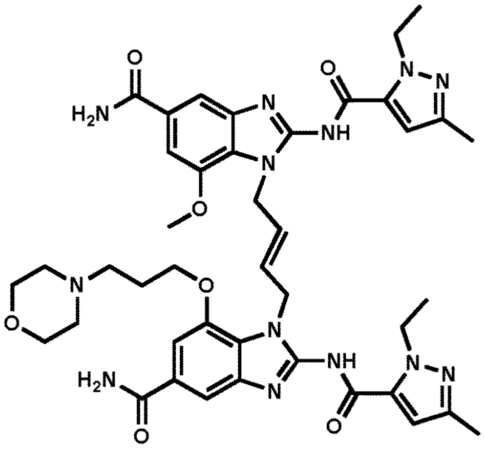

ou d'un tautomère de celui-ci,
ou d'un sel pharmaceutiquement acceptable de celui-ci ;

b) d'un agent de charge ou d'une combinaison d'agents de charge ; et
c) d'un agent solubilisant choisi parmi : l'acide aspartique, l'acide acétique, l'acide glutamique, l'acide méthane sulfonique, l'acide lactique et l'acide glucuronique ;
d) d'eau ; et

2) optionnellement d'évaporation ou de lyophilisation du mélange pour former une poudre ou un gâteau.

**16.** Formulation pharmaceutique telle que définie selon l'une quelconque des revendications 1 à 12, pour une utilisation dans le traitement d'une maladie dans laquelle la modulation de STING (stimulateur des gènes de l'interféron) est bénéfique, choisie parmi l'inflammation, les maladies allergiques et auto-immunes, les maladies infectieuses, le virus de l'hépatite C (HCV), le virus de l'hépatite B (HBV), la grippe, les verrues cutanées, la sclérose en plaques, le virus de l'immunodéficience humaine (VIH), le SIDA, le cancer et les syndromes précancéreux.

**17.** Formulation pharmaceutique selon la revendication 16, dans laquelle l'utilisation est pour le traitement du cancer choisi parmi : cerveau (gliomes), glioblastomes, astrocytomes, glioblastome multiforme, syndrome de Bannayan-Zonana, maladie de Cowden, maladie de Lhermitte-Duclos, tumeur de Wilms, sarcome d'Ewing, rhabdomyosarcome, épendymome, médulloblastome, tête et cou, rein, foie, mélanome, ovarien, pancréatique, adénocarcinome, adénocarcinome canalaire, carcinome adénosquameux, carcinome à cellules acineuses, glucagonome, insulinome, prostate, sarcome, ostéosarcome, tumeur osseuse à cellules géantes, thyroïde, leucémie lymphoblastique à cellules T, leucémie myéloïde chronique, leucémie lymphoïde chronique, leucémie à cellules chevelues, leucémie aiguë lymphoblastique, leucémie aiguë myéloïde, leucémie chronique à neutrophiles, leucémie aiguë lymphoblastique T, plasmocytome, leucémie de type immunoblastique à grandes cellules, leucémie à cellules du manteau, myélome multiple, leucémie mégacaryoblastique, myélome multiple, leucémie aigüe mégacaryocytaire, leucémie promyélocytaire, érythroleucémie, lymphome malin, lymphome hodgkinien, lymphome non hodgkinien, lymphome lymphoblastique à cellules T, lymphome de Burkitt, lymphome folliculaire, neuroblastome, cancer de la vessie, cancer urothélial, cancer de la vulve, cancer du col de l'utérus, cancer de l'endomètre, cancer du rein, mésothéliome, cancer de l'oesophage, cancer des glandes salivaires, cancer hépatocellulaire, cancer gastrique, cancer nasopharyngé, cancer buccal, cancer de la bouche, GIST (tumeur stromale gastro-intestinale) et cancer des testicules.

**18.** Formulation pharmaceutique selon la revendication 16, dans laquelle l'utilisation est pour le traitement du cancer choisi parmi : cancer du poumon non à petites cellules (CPNPC), vessie, carcinome à cellules squameuses de la tête et du cou, col de l'utérus, endomètre, ovaire, pancréas, cancer colorectal à microsatellites stables, cancer colorectal à instabilité élevée des microsatellites, estomac, endo oesophagien, jonction gastro-oesophagienne, oesophage, squameux, leucémie aiguë myéloïde et cancer du sein triple négatif.

**19.** Formulation pharmaceutique selon la revendication 16, dans laquelle l'utilisation est pour le traitement des syndromes précancéreux choisis parmi : néoplasie cervicale intraépithéliale, gammapathie monoclonale de signification indéterminée (GMSI), syndrome myélodysplasique, anémie aplasique, lésions cervicales, naevus (prémélanome), néoplasie intraépithéliale (intracanalaire) prostatique (PIN), carcinome canalaire in situ (CCIS), polypes du côlon et

hépatite sévère ou cirrhose.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017051945 A
- WO 2017175147 A
- WO 2018057738 A
- WO 2019069275 A


### Non-patent literature cited in the description


- **S. H. YALKOWSKY.** *Journal of Pharmaceutical Sciences,* July 1998, vol. 87 (7), 787-796